(19) Europäisches Patentamt
European Patent Office
Office européen des brevets

(11) EP 0 927 721 A1

(12) EUROPÄISCHE PATENTANMELDUNG

(43) Veröffentlichungstag:
**07.07.1999 Patentblatt 1999/27**

(51) Int. Cl.[6]: **C07F 9/40**, A61K 31/66, C07F 9/53, C07C 401/00

(21) Anmeldenummer: **97250374.2**

(22) Anmeldetag: **17.12.1997**

(84) Benannte Vertragsstaaten:
**AT BE CH DE DK ES FI FR GB GR IE IT LI LU MC NL PT SE**
Benannte Erstreckungsstaaten:
**AL LT LV MK RO SI**

(71) Anmelder:
**SCHERING AKTIENGESELLSCHAFT**
**13342 Berlin (DE)**

(72) Erfinder:
- **Steinmeyer, Andreas, Dr.**
  **13581 Berlin (DE)**
- **Neef, Günter, Dr.**
  **10711 Berlin (DE)**
- **Kirsch, Gerald, Dr.**
  **14199 Berlin (DE)**
- **Schwarz, Katica**
  **10585 Berlin (DE)**
- **Wiesinger, Herbert, Dr.**
  **10781 Berlin (DE)**
- **Haberey, Martin, Dr.**
  **12169 Berlin (DE)**
- **Fähnrich, Marianne, Dr.**
  **13409 Berlin (DE)**
- **Langer, Gernot, Dr.**
  **10551 Berlin (DE)**

(54) **Neue Vitamin D-Derivate mit Phosphoratomen in den Seitenketten, Zwischenprodukte bei ihrer Herstellung und die Verwendung zur Herstellung von Arzneimitteln**

(57) Die Erfindung betrifft neue Vitamin D-Derivate der allgemeinen Formel **I**

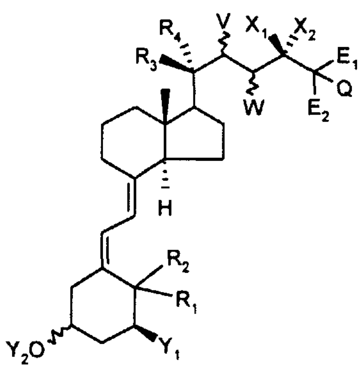

**I**

Zwischenprodukte des Verfahrens zu ihrer Herstellung sowie die Verwendung zur Herstellung von Arzneimitteln.

EP 0 927 721 A1

**Beschreibung**

Die Erfindung betrifft neue Vitamin D-Derivate der allgemeinen Formel **I**

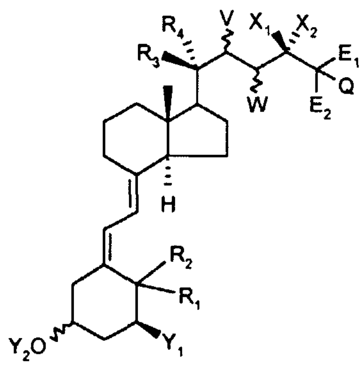

**I**

Zwischenprodukte des Verfahrens zu ihrer Herstellung sowie die Verwendung zur Herstellung von Arzneimitteln.

**Stand der Technik**

Die natürlichen Vitamine $D_2$ und $D_3$ sind an sich biologisch inaktiv und werden erst nach Hydroxylierung am C-Atom 25 in der Leber und am C-Atom 1 in der Niere in biologisch aktive Metaboliten [1α,25-Dihydroxyvitamin $D_3$ (Calcitriol) bzw. - $D_2$] umgewandelt. Die Wirkung der aktiven Metaboliten besteht in der Regulation der Calcium- und Phosphatkonzentration im Serum; sie wirken einem Absinken der Calciumkonzentration im Serum entgegen, indem sie die Calciumabsorption im Darm erhöhen und unter bestimmten Umständen die Calciummobilisation aus dem Knochen fördern. Figur 1 zeigt die Struktur einiger bekannter Vitamin D-Derivate.

Neben ihrer ausgeprägten Wirkung auf den Calcium- und Phosphatstoffwechsel besitzen die aktiven Metaboliten von Vitamin $D_2$ und $D_3$ und seine synthetischen Abkömmlinge eine proliferationshemmende und differenzierungsstimulierende Wirkung auf Tumorzellen und normale Zellen, wie zum Bespiel Hautzellen. Weiterhin wurde eine ausgeprägte Wirkung auf Zellen des Immunsystems (Hemmung der Proliferation und Interleukin 2-Synthese von Lymphocyten, Steigerung der Cytotoxizität und Phagocytose in vitro von Monocyten) gefunden, die sich in einer immunmodulatorischen Wirkung äußert, schließlich wird infolge einer fordernden Wirkung auf knochenbildende Zellen eine vermehrte Knochenbildung bei normalen und osteoporotischen Ratten gefunden [R. Bouillon et al. "Short term course of 1,25-$(OH)_2D_3$ stimulates osteoblasts but not osteoclasts" . *Calc. Tissue Int.* **49,** 168(1991)]

Alle Wirkungen werden durch Bindung an den Vitamin D-Rezeptor vermittelt. Infolge der Bindung wird die Aktivität von spezifischen Genen reguliert.

Bei Anwendung der biologisch aktiven Metaboliten von Vitamin $D_2$ und $D_3$ wird eine toxische Wirkung auf den Calciumstoffwechsel hervorgerufen (Hypercalcämie).

Durch strukurelle Manipulationen der Seitenkette können therapeutisch nutzbare Wirkqualitäten von der unerwünschten hypercalcämischen Aktivität abgetrennt werden. Eine geeignete Strukturvariante ist die Einführung einer 24-Hydroxy-Gruppe.

In 24-Stellung hydroxylierte 1α-Cholecalciferole gehen bereits aus der DE 25 26 981 hervor. Sie besitzen eine geringere Toxizität als das entsprechende nicht-hydroxylierte 1α-Cholecalciferol. Darübertunaus sind 24-Hydroxy-Derivate in folgenden Patentanmeldungen beschrieben:
DE 39 33 034, DE 40 03 854, DE 40 34 730, EP 0 421 561, EP 0 441 467, WO 87/00834, WO 91/12238.

Schließlich werden in der WO 94/07853 an C-24 hydroxylierte 25-Carbonsäure-Derivate von Calcitriol beschrieben, die ein günstigeres Wirkspektrum als Calcitriol aufweisen. Entsprechendes gilt auch für neue Vitamin D-Derivate mit anderen Substituenten an C-25 (WO 97/00242). Während die Fähigkeit zur Auslösung einer Hypercalcämie deutlich abgeschwächt ist, bleiben die proliferationshemmenden und differenzierungsstimuliernden Wirkungen erhalten. In der Regel führt die Einführung der 24-Hydroxylgruppe jedoch zur metabolischen Destabilisierung der Derivate, insbesondere wenn sich in der Nachbarstellung ein Cyclopropylring befindet. Aus diesem Grunde sind diese Verbindungen nur bedingt zur systemischen Applikation geeignet.

Es besteht daher Bedarf an neuen Vitamin D-Derivaten, die ein ähnlich günstiges oder verbessertes Wirkspektrum, wie die im Stand der Technik beschriebenen Verbindungen (insbesondere WO 94/07853 und WO 97/00242) aufweisen, aber durch eine höhere metabolische Stabilität besser zur systemischen Applikation geeignet sind.

Der vorliegenden Patentanmeldung liegt daher die Aufgabe zugrunde, derartige Vitamin D-Derivate zur Verfügung zu stellen. Die Lösung dieser Aufgabe erfolgt durch die in den Patentansprüchen offenbarten Verbindungen.

Die vorliegende Erfindung betrifft daher Vitamin D-Derivate der allgemeinen Formel **I**,

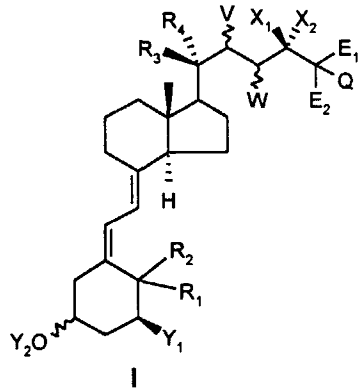

**I**

worin

$Y_1$ ein Wasserstoffatom, eine Hydroxylgruppe, ein Fluor-, Chlor- oder Bromatom oder eine Gruppe $-O(CO)R_5$, worin
$R_5$ ein aliphatischer oder aromatischer Rest mit 1 bis 12 C-Atomen ist,

$Y_2$ ein Wasserstoffatom oder eine Gruppe $-(CO)R_6$, worin
$R_6$ ein aliphatischer oder aromatischer Rest mit 1 bis 12 C-Atomen ist

und die Gruppe $Y_2O$ sowohl in der natürlich vorkommenden (3β) als auch der epimeren (3α) Situation vorliegen kann,

$R_1$ und $R_2$ je ein Wasserstoffatom oder gemeinsam eine exocyclische Methylengruppe,

$R_3$ und $R_4$ unabhängig voneinander ein Wasserstoffatom, ein Chlor- oder Fluoratom, eine Alkylgruppe mit 1 bis 4 Kohlenstoffatomen, gemeinsam eine Methylengruppe oder gemeinsam mit dem quartären Kohlenstoffatom 20 einen 3-7-gliedrigen, gesättigten oder ungesättigten carbocyclischen Ring,

V und W zusammen eine *E*-Doppelbindung oder V eine Hydroxylgruppe und W ein Wasserstoffatom oder V und W jeweils Wasserstoffatome,

$X_1$ und $X_2$ gemeinsam ein Carbonylgruppe oder je ein Wasserstoffatom oder $X_2$ ein Wasserstoffatom und $X_1$ eine Hydroxylgruppe oder eine Gruppe $-O(CO)R_7$, worin
$R_7$ ein aliphatischer oder aromatischer Rest mit 1 bis 12 C-Atomen ist,
oder $X_1$ ein Wasserstoffatom und $X_2$ eine Hydroxylgruppe oder eine Gruppe $-O(CO)R_8$, worin
$R_8$ ein aliphatischer oder aromatischer Rest mit 1 bis 12 C-Atomen ist,
oder

$X_1$ und $E_2$ eine Doppelbindung und gleichzeitig $X_2$ ein Wasserstoffatom oder eine Gruppe O-Z,
wobei Z ein aliphatischer oder aromatischer Rest mit 1 bis 12 C-Atomen, eine aliphatische oder aromatische Acylgruppe mit 1 bis 12 C-Atomen oder eine Gruppe $E_2$,

$E_1$ eine Gruppe $PO(OR_9)_2$, eine Gruppe $PO(N(R_9)_2)_2$, eine Gruppe $PO(R_9)_2$ oder eine Gruppe $CO_2R_9$worin
$R_9$ ein Wasserstoffatom oder ein aliphatischer oder aromatischer Rest mit 1 bis 12C-Atomen ist,

$E_2$ eine Gruppe $PO(OR_9)_2$, eine Gruppe $PO(N(R_9)_2)_2$, eine Gruppe $PO(R_9)_2$, eine Gruppe $CO_2R_9$ oder ein Wasserstoffatom,

Q ein Wasserstoffatom, ein aliphatischer oder aromatischer Rest mit 1 bis 12 C-Atomen oder eine Hydroxylgruppe, eine Gruppe $-O(CO)R_{10}$, ein Fluor, Chlor oder Bromatom, eine Aminogruppe oder eine Gruppe $NHR_{10}$ oder $N(R_{10})_2$, worin
$R_{10}$ ein aliphatischer oder aromatischer Rest mit 1 bis 12 C-Atomen ist,

bedeuten.

Die Erfindung betrifft ferner Verfahren zur Herstellung der erfindungsgemäßen Verbindungen, Zwischenprodukte des Herstellungsverfahrens sowie die Verwendung der erfindungsgemäßen Verbindungen zur Herstellung von Arzneimitteln.

Besonders vorteilhafte Ausführungsformen der Erfindung sind Gegenstand der Unteransprüche.

Die Gruppen $E_1$ und $E_2$ stehen für Phosphonsäure- oder Carbonsäurederivate oder für Phosphinoxidderivate. Der Begriff Phosphonsäure- oder Carbonsäurederivate umfaßt die freien Phosphon- oder Carbonsäuren ($-PO_3H_2$, $-CO_2H$), Phosphon- oder Carbonsäureester ($-PO_3(R_9)_2$, $-CO_2R_9$), Phosphon- oder Carbonsäureamide (z.B. $-PO_3(N(R_9)_2)_2$, $-CO_2(NR_9)_2$, $-PO_3(NHR_9)_2$, $-CO_2NHR_9$), aber auch Phosphonsäuremonoester oder -amide. Erfindungsgemäß bevorzugt sind Verbindungen in denen mindestens eine phosphorhaltige Gruppe enthalten ist. Besonders bevorzugt sind Verbindungen, in denen beide Gruppen ($E_1$ und $E_2$) für Phosphonsäurederivate stehen.

Die Gruppen $R_3$ und $R_4$ können unabhängig voneinander ein Wasserstoffatom, ein Chlor- oder Fluoratom, eine Alkylgruppe mit 1 bis 4 Kohlenstoffatomen (Methyl-, Ethyl,- n-Propyl-, i-Propyl, n-Butyl-, i-Butyl-, t-Butyl-), gemeinsam eine Methylengruppe oder gemeinsam mit dem quartären Kohlenstoffatom 20 einen 3-7-gliedrigen, gesättigten oder ungesättigten carbocyclischen Ring bedeuten.

Für $R_3$ und $R_4$ gelten die folgenden bevorzugten Kombinationen: $R_3$= H, $R_4$= Methyl oder $R_3$= Methyl, $R_4$=H; $R_3$= F, $R_4$= Methyl oder $R_3$= Methyl, $R_4$=F; $R_3$, $R_4$ = Methyl; $R_3$ und $R_4$ bilden zusammen eine Methylengruppe oder gemeinsam mit dem tertiären Kohlenstoffatom 20 einen Cyclopropylring.

Die optionalen Reste $R_5$, $R_6$, $R_7$, $R_8$, $R_9$ und $R_{10}$ sind organische Gruppen mit 1 bis 12 C-Atomen. Diese Reste können gesättigt oder ungesättigt, verzweigt oder unverzweigt, acyclisch, carbocyclisch oder heterocyclisch sein. Beispiele für die Reste $R_5$, $R_6$, $R_7$, $R_8$, $R_9$ und $R_{10}$ sind Methyl-, Ethyl-, Propyl-, i-Propyl-, Butyl-oder Phenylgruppen. Möglich sind aber auch die Reste natürlich vorkommender Aminosäuren, wie z.B. $-CH_2-CH(CH_3)_2$, $-CH_2-Ph$, $-CH_2OH$, $-CH(OH)-CH_3$, $-CH_2SH$, $-CH_2-SCH_3$, $-CH_2CO_2H$, $-CH_2CH_2-CO_2H$, $-(CH_2)_4-NH_2$, $-(CH_2)_3-C(NH)NH_2$, aber auch die Reste der Aminosäuren Tryptophan, Tyrosin oder Histamin.

Die bevorzugten Reste leiten sich von $C_1$- bis $C_9$-, insbesondere $C_2$- bis $C_5$-Alkancarbonsäuren wie beispielsweise Essigsäure, Propionsäure, Buttersäure oder Pivaloylsäure ab. Unter den aromatischen Gruppen sind die Phenylgruppe und substituierte Phenylgruppen bevorzugt.

Die Gruppen V und W sind jeweils Wasserstoffatome oder bilden entweder zusammen eine *E*-Doppelbindung oder V ist eine Hydroxylgruppe und W ein Wasserstoffatom. Die Möglichkeiten für das betreffende Strukturelement sind im Folgenden abgebildet:

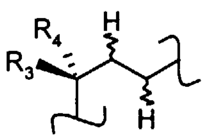

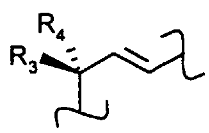

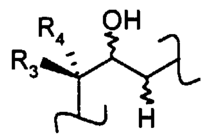

Weiterhin bevorzugt ist die Konstellation, daß V und W und $X_1$ und $E_2$ jeweils eine *E*-Doppelbindung bedeuten, während $X_2$ und Q Wasserstoffatome sind.

Außerdem bevorzugt ist die Konstellation, daß V und W zusammen eine *E*-Doppelbindung bilden, $X_1$ und $E_2$ eine *E*- oder Z-Doppelbindung bilden, $X_2$ eine Gruppe -OZ ist und Q ein Wasserstoffatom bedeutet.

Von den erfindungsgemäßen Verbindungen der allgemeinen Formel **I** sind die folgenden Verbindungen ganz besonders bevorzugt:

(5*Z*,7*E*,22*E*)-(1*S*,3*R*)-(1,3-Dihydroxy-24-oxo-24a-homo-9, 10-secochola-5,7,10(19),22-tetraen-24a-yl)phosphonsäuredimethylester

(5*Z*,7*E*,22*E*))-(1*S*,3*R*,24*S*)-(1,3,24-Trihydroxy-24a-homo-9,10-secochola-5,7,10(19),22-tetraen-24a-yl)phosphonsäuredimethylester

(5*Z*,7*E*,22*E*)-(1*S*,3*R*,24*R*)-(1,3,24-Trihydroxy-24a-homo-9,10-secochola-5,7,10(19),22-tetraen-24a-yl)phosphonsäuredimethylester

(5*Z*,7*E*,22*E*)-(1*S*,3*R*)-(1,3-Dihydroxy-24-oxo-24a-homo-9,10-secochola-5,7,10(19),22-tetraen-24a-yl)phosphonsäurediethylester

(5*Z*,7*E*,22*E*)-(1*S*,3*R*,24*S*)-(1,3,24-Trihydroxy-24a-homo-9,10-secochola-5,7,10(19),22-tetraen-24a-yl)phosphonsäurediethylester

(5*Z*,7*E*,22*E*)-(1*S*,3*R*,24*R*)-(1,3,24-Trihydroxy-24a-homo-9,10-secochola-5,7,10(19),22-tetraen-24a-yl)phosphonsäurediethylester

(5*Z*,7*E*,22*E*)-(1*S*,3*R*)-(1,3-Dihydroxy-24-oxo-24a-homo-9,10-secochola-5,7,10(19),22-tetraen-24a-yl)phosphonsäurebis(1-methylethyl)ester

(5*Z*,7*E*,22*E*)-(1*S*,3*R*,24*S*)-(1,3,24-Trihydroxy-24a-homo-9,10-secochola-5,7,10(19),22-tetraen-24a-yl)phosphonsäurebis(1-methylethyl)ester

(5*Z*,7*E*,22*E*)-(1*S*,3*R*,24*R*)-(1,3,24-Trihydroxy-24a-homo-9,10-secochola-5,7,10(19),22-tetraen-24a-yl)phosphonsäurebis(1-methylethyl)ester

(5*Z*,7*E*,22*E*)-(1*S*,3*R*)-(1,3-Dihydroxy-24-oxo-24a-homo-9,10-secochola-5,7,10(19),22-tetraen-24a-yl)phosphonsäuredipropylester

(5*Z*,7*E*,22*E*)-(1*S*,3*R*,24*S*)-(1,3,24-Trihydroxy-24a-homo-9,10-secochola-5,7,10(19),22-tetraen-24a-yl)phosphonsäuredipropylester

(5*Z*,7*E*,22*E*)-(1*S*,3*R*,24*R*)-(1,3,24-Trihydroxy-24a-homo-9,10-secochola-5,7,10(19),22-tetraen-24a-yl)phosphonsäuredipropylester

(5*Z*,7*E*,22*E*)-(1*S*,3*R*)-(1,3-Dihydroxy-24-oxo-24a-homo-9,10-secochola-5,7,10(19),22-tetraen-24a-yl)phosphonsäuredibutylester

(5*Z*,7*E*,22*E*)-(1*S*,3*R*,24*S*)-(1,3,24-Trihydroxy-24a-homo-9,10-secochola-5,7,10(19),22-tetraen-24a-yl)phosphonsäuredibutylester

(5*Z*,7*E*,22*E*)-(1*S*,3*R*,24*R*)-(1,3,24-Trihydroxy-24a-homo-9,10-secochola-5,7,10(19),22-tetraen-24-ayl)phosphonsäuredibutylester

(5*Z*,7*E*,22*E*)-(1*S*,3*R*)-(1,3-Dihydroxy-24-oxo-24a-homo-9,10-secochola-5,7,10(19),22-tetraen-24a-yl)phosphonsäuredipentylester

(5*Z*,7*E*,22*E*)-(1*S*,3*R*,24*S*)-(1,3,24-Trihydroxy-24a-homo-9,10-secochola-5,7,10(19),22-tetraen-24a-yl)phosphonsäuredipentylester

(5*Z*,7*E*,22*E*)-(1*S*,3*R*,24*R*)-(1,3,24-Trihydroxy-24a-homo-9,10-secochola-5,7,10(19),22-tetraen-24a-yl)phosphonsäuredipentylester

(5*Z*,7*E*,22*E*)-(1*S*,3*R*)-(1,3-Dihydroxy-24-oxo-24a-homo-9,10-secochola-5,7,10(19),22-tetraen-24a-yl)phosphonsäurediphenylester

(5*Z*,7*E*,22*E*)-(1*S*,3*R*,24*S*)-(1,3,24-Trihydroxy-24a-homo-9,10-secochola-5,7,10(19),22-tetraen-24a-yl)phosphonsäurediphenylester

(5*Z*,7*E*,22*E*)-(1*S*,3*R*,24*R*)-(1,3,24-Trihydroxy-24a-homo-9,10-secochola-5,7,10(19),22-tetraen-24a-yl)phosphonsäurediphenylester

(5*Z*,7*E*,22*E*)-(1*S*,3*R*)-(1,3-Dihydroxy-24-oxo-24a-homo-9,10-secochola-5,7,10(19),22-tetraen-24a,24a-diyl)bis[phosphonsäuredimethylester]

(5*Z*,7*E*,22*E*)-(1*S*,3*R*)-(1,3-Dihydroxy-24-oxo-24a-homo-9,10-secochola-5,7,10(19),22-tetraen-24a,24a-diyl)bis[phosphonsäurediethylester]

(5*Z*,7*E*,22*E*)-(1*S*,3*R*)-(1,3-Dihydroxy-24-oxo-24a-homo-9,10-secochola-5,7,10(19),22-tetraen-24a,24a-diyl)bis[phosphonsäuredipropylester]

(5*Z*,7*E*,22*E*)-(1*S*,3*R*)-(1,3-Dihydroxy-24-oxo-24a-homo-9,10-secochola-5,7,10(19),22-tetraen-24a,24a-diyl)bis[phosphonsäuredibutylester]

(5*Z*,7*E*,22*E*)-(1*S*,3*R*)-(1,3-Dihydroxy-24-oxo-24a-homo-9,10-secochola-5,7,10(19),22-tetraen-24a,24a-diyl)bis[phosphonsäurebis(1-methylethyl)ester]

(5*Z*,7*E*,22*E*)-(1*S*,3*R*)-(1,3-Dihydroxy-24-oxo-24a-homo-9,10-secochola-5,7,10(19),22-tetraen-24a,24a-diyl)bis[phosphonsäurediphenylester]

(5*Z*,7*E*,22*E*,24*E*)-(1*S*,3*R*)-(1,3-Dihydroxy-24a-homo-9,10-secochola-5,7,10(19),22,24-pentaen-24a-yl)phosphonsäuredimethylester

(5*Z*,7*E*,22*E*,24*E*)-(1*S*,3*R*)-(1,3-Dihydroxy-24a-homo-9,10-secochola-5,7,10(19),22,24-pentaen-24a-yl)phosphonsäurediethylester

(5*Z*,7*E*,22*E*,24*E*)-(1*S*,3*R*)-(1,3-Dihydroxy-24a-homo-9,10-secochola-5,7,10(19),22,24-pentaen-24a-yl)phosphonsäuredipropylester

(5*Z*,7*E*,22*E*,24*E*)-(1*S*,3*R*)-(1,3-Dihydroxy-24a-homo-9,10-secochola-5,7,10(19),22,24-pentaen-24a-yl)phosphonsäuredibutylester

(5*Z*,7*E*,22*E*,24*E*)-(1*S*,3*R*)-(1,3-Dihydroxy-24a-homo-9,10-secochola-5,7,10(19),22,24-pentaen-24a-yl)phosphonsäurebis(1-methylethyl)ester

(5*Z*,7*E*,22*E*)-(1*S*,3*R*)-24-(Diphenylphosphinyl)-9,10-secochola-5,7,10(19),22-tetraen-1,3-diol

(5*Z*,7*E*,22*E*)-(1*S*,3*R*)-24-(Dimethylphosphinyl)-9,10-secochola-5,7,10(19),22-tetraen-1,3-diol

(5*Z*,7*E*,22*E*)-(1*S*,3*R*)-24-(Diethylphosphinyl)-9,10-secochola-5,7,10(19),22-tetraen-1,3-diol

(5*Z*,7*E*,22*E*)-(1*S*,3*R*)-24-(Dipropylphosphinyl)-9,10-secochola-5,7,10(19),22-tetraen-1,3-diol

(5*Z*,7*E*,22*E*)-(1*S*,3*R*)-24-(Dibutylphosphinyl)-9,10-secochola-5,7,10(19),22-tetraen-1,3-diol

(5*Z*,7*E*,22*E*,24*E*)-(1*S*,3*R*)-Phosphorsäure[1,3-dihydroxy-24a-(dimethoxy)phosphinyl-24a-homo-9,10-secochola-5,7,10(19),22,24-pentaen-24-yl]dimethylester

(5*Z*,7*E*,22*E*,24*Z*)-(1*S*,3*R*)-Phosphorsäure[1,3-dihydroxy-24a-(dimethoxy)phosphinyl-24a-homo-9,10-secochola-5,7,10(19),22,24-pentaen-24-yl]dimethylester

(5*Z*,7*E*,22*E*,24*E*)-(1*S*,3*R*)-Phosphorsäure[24a-(diethoxy)phosphinyl-1,3-dihydroxy-24a-homo-9,10-secochola-5,7,10(19),22,24-pentaen-24-yl]diethylester

(5*Z*,7*E*,22*E*,24*Z*)-(1*S*,3*R*)-Phosphorsäure[24a-(diethoxy)phosphinyl-1,3-dihydroxy-24a-homo-9,10-secochola-5,7,10(19),22,24-pentaen-24-yl]diethylester

(5*Z*,7*E*,22*E*,24*E*)-(1*S*,3*R*)-Phosphorsäure[1,3-dihydroxy-24a-(dipropoxy)phosphinyl-24a-homo-9,10-secochola-5,7,10(19),22,24-pentaen-24-yl]dipropylester

(5*Z*,7*E*,22*E*,24*Z*)-(1*S*,3*R*)-Phosphorsäure[1,3-dihydroxy-24a-(dipropoxy)phosphinyl-24a-homo-9,10-secochola-5,7,10(19),22,24-pentaen-24-yl]dipropylester

(5*Z*,7*E*,22*E*,24*E*)-(1*S*,3*R*)-Phosphorsäure[24a(dibutoxy)phosphinyl-1,3-dihydroxy-24a-homo-9,10-secochola-5,7,10(19),22,24-pentaen-24-yl]dibutylester

(5*Z*,7*E*,22*E*,24*Z*)-(1*S*,3*R*)-Phosphorsäure[24a-(dibutoxy)phosphinyl-1,3-dihydroxy-24a-homo-9,10-secochola-5,7,10(19),22,24-pentaen-24-yl]dibutylester

(5*Z*,7*E*,22*E*,24*E*)-(1*S*,3*R*)-Phosphorsäure[24a-[bis(1-methylethoxy)phosphinyl]-1,3-dihydroxy-24a-homo-9,10-secochola-5,7,10(19),22,24-pentaen-24-yl]bis(1-methylethyl)ester

(5*Z*,7*E*,22*E*,24*Z*)-(1*S*,3*R*)-Phosphorsäure[24a[bis(1-methylethoxy)phosphinyl]-1,3-dihydroxy-24a-homo-9,10-secochola-5,7,10(19),22,24-pentaen-24-yl]bis(1-methylethyl)ester

Die erfindungsgemäßen Substanzen besitzen eine deutlich höhere metabolische Stabilität als die strukturell verwandten Verbindungen des Standes der Technik und eignen sich daher in besonderer Weise für systemische Applikationen.

Gegenüber den strukturell verwandten Verbindungen des Standes der Technik zeichnen sich einige der erfindungsgemäßen Substanzen ferner dadurch aus, daß sie eine starke Wirkung auf die Zelldifferenzierung zeigen, wobei die Wirkung auf den Calciumhaushalt nicht zunimmt.

Überraschenderweise wurde für einige der erfindungsgemäßen Substanzen gefunden, daß sie gezielt den Aufbau von neuem Knochenmaterial fördern, ohne gleichzeitig einen Anstieg der Serumcalciumspiegel zu bewirken.

Andere der erfindungsgemäßen Substanzen dagegen weisen ein antagonistisches oder partialagonistisches Wirkprofil auf, das neue Anwendungen ermöglicht.

**Bestimmung der biologischen Aktivität**

Die Vitamin D-Aktivität der erfindungsgemäßen Substanzen wird mittels des Calcitriol-Rezeptortests bestimmt. Er wird unter Verwendung eines Proteinextraktes aus dem Darm von jungen Schweinen durchgeführt. Rezeptorhaltiger Proteinextrakt wird mit $^3$H-Calcitriol (5 x $10^{-10}$ mol/l) in einem Reaktionsvolumen von 0,27 ml in Abwesenheit und in Anwesenheit der Prüfsubstanzen für zwei Stunden bei 4°C in einem Teströhrchen inkubiert. Zur Trennung von freiem und rezeptorgebundenem Calcitriol wird eine Charcoal-Dextran-Absorption durchgeführt. Dazu werden 250 µl einer Charcoal-Dextran-Suspension jedem Teströhrchen zugeführt und bei 4°C für 20 Minuten inkubiert. Anschließend werden die Proben bei 10 000 g 5 Minuten bei 4°C zentrifugiert. Der Überstand wird dekantiert und nach einstündiger Äquilibrierung in Picofluor 15 ™ in einem β-Zähler gemessen.

Die mit verschiedenen Konzentrationen der Prüfsubstanz sowie der Referenzsubstanz (unmarkiertes Calcitriol) bei konstanter Konzentration der Bezugssubstanz ($^3$H-Calcitriol) erhaltenen Kompetitionskurven werden in Beziehung zueinander gesetzt und ein Kompetitionsfaktor (KF) ermittelt.

Er ist definiert als Quotient aus den Konzentrationen der jeweiligen Prüfsubstanz und der Referenzsubstanz, die für 50%ige Kompetition erforderlich sind:

$$KF=\frac{\text{Konzentration Prüfsubstanz bei 50\% Kompetition}}{\text{Konzentration Referenzsubstanz bei 50\% Kompetition}}$$

Zur Bestimmung der akuten hypercalcämischen Wirkung verschiedener Calcitriolderivate wird nachfolgend beschriebener Test durchgeführt:

Die Wirkung von Kontrolle (Lösungsgrundlage), Referenzsubstanz (1,25-Dihydroxyvitamin $D_3$ = Calcitriol) und Testsubstanz wird jeweils nach einmaliger subcutaner Applikation in Gruppen von 10 gesunden männlichen Ratten (140-170 g) getestet. Die Ratten werden während der Versuchszeit in speziellen Käfigen zur Bestimmung der Exkretion von Wasser und Mineralstoffen gehalten. Der Harn wird in 2 Fraktionen (0-16 h und 16-22 h) gesammelt. Eine orale Calciumgabe (0.1 mM Calcium in 6,5% Alpha-Hydroxypropylcellulose, 5 ml/Tier) ersetzt zum Zeitpunkt 16 h die durch Futterentzug fehlende Calciumaufnahme. Zu Versuchsende werden die Tiere durch Dekapitieren getötet und für die

Bestimmung der Serum-Calciumwerte entblutet. Für die primäre Screen-Untersuchung in vivo wird eine einzelne Standarddosis (200 µg/kg) getestet. Für ausgewählte Substanzen wird das Ergebnis durch Erstellung einer Dosis-Wirkungs-Beziehung abgesichert.

Eine hypercalcämische Wirkung zeigt sich in im Vergleich zur Kontrolle erhöhten Serum-Calciumspiegel-Werten.

Die Signifikanz auftretender Unterschiede zwischen Substanzgruppen und Kontrollen sowie zwischen Testsubstanz und Referenzsubstanz werden mit geeigneten statistischen Verfahren abgesichert. Das Ergebnis wird als Dosisrelation DR (DR = Faktor Testsubstanzdosis/Referenzsubstanzdosis für vergleichbare Wirkungen) angegeben.

Die differenzierungsstimulierende Wirkung von Calcitriolanaloga wird ebenfalls quantitativ erfaßt.

Es ist literaturbekannt [Mangelsdorf, D.J. et al., *J Cell. Biol.* <u>**98**</u>, 391 (1984)], daß die Behandlung humaner Leukämiezellen (Promyelocytenzellinie HL 60) in vitro mit Calcitriol die Differenzierung der Zellen zu Makrophagen induziert.

HL 60-Zellen werden in Gewebekulturmedium (RPMI 10% fetales Kälberserum) bei 37°C in einer Atmosphäre 5% $CO_2$ in Luft kultiviert.

Zur Substanztestung werden die Zellen abzentrifugiert und 2,0 x $10^5$ Zellen/ml in phenolrotfreiem Gewebekulturmedium aufgenommen. Die Testsubstanzen werden in Ethanol gelöst und mit Gewebekulturmedium ohne Phenolrot auf die gewünschte Konzentration verdünnt. Die Verdünnungsstufen werden mit der Zellsuspension in einem Verhältnis von 1:10 gemischt und je 100 µl dieser mit Substanz versetzten Zellsuspension in eine Vertiefung einer 96-Loch-Platte pipettiert. Zur Kontrolle wird eine Zellsuspension analog mit dem Lösungsmittel versetzt.

Nach Inkubation über 96 Stunden bei 37°C in 5% $CO_2$ in Luft wird in jede Vertiefung der 96-Loch-Platte zu der Zellsuspension 100 µl einer NBT-TPA-Lösung (Nitroblautetrazolium (NBT), Endkonzentration im Ansatz 1 mg/ml, Tetradecanoylphorbolmyrisiat-13-acetat (TPA), Endkonzentration im Ansatz 2 x $10^{-7}$ mol/l) pipettiert.

Durch Inkubation über 2 Stunden bei 37°C und 5% $CO_2$ in Luft wird infolge der intrazellulären Sauerstoffradikalfieisetzung, stimuliert durch TPA, in den zu Makrophagen differenzierten Zellen NBT zu unlöslichem Formazan reduziert.

Zur Beendigung der Reaktion werden die Vertiefungen der 96-Loch-Platte abgesaugt und die Zellen durch Zugabe von Methanol am Plattenboden fixiert und nach Fixation getrocknet. Zur Lösung der gebildeten intrazellulären Formazankristalle werden in jede Vertiefung 100 µl Kaliumhydroxid (2 mol/l) und 100 µl Dimethylsulfoxid pipettiert und 1 Minute ultrabeschallt. Die Konzentration von Formazan wird spektralphotometrisch bei 650 nm gemessen.

Als Maß für die Differenzierungsinduktion der HL 60-Zellen zu Makrophagen gilt die Konzentration an gebildetem Formazan. Das Ergebnis wird als Dosisrelation (DR = Faktor Testsubstanzdosis/Referenzsubstanzdosis für vergleichbare halbmaximale Wirkungen) angegeben.

Die Ergebnisse des Calcitriol-Rezeptortests sowie der Bestimmung der Dosisrelation der Differenzierungsinduktion von HL 60-Zellen und der Dosisrelation für Hypercalcämie sind nachfolgend zusammengefaßt:

Beispiele für Testsubstanzen:

(5*Z*,7*E*,22*E*)-(1*S*,3*R*)-(1,3-Dihydroxy-24-oxo-24a-homo-9,10-secochola-5,7,10(19),22-tetraen-24a-yl)phosphonsäuredimethylester **5**

(5*Z*,7*E*,22*E*)-(1*S*,3*R*)-(1,3-Dihydroxy-24-oxo-24a-homo-9,10-secochola-5,7,10(19),22-tetraen-24a-yl)phosphonsäurediethylester **9**

(5*Z*,7*E*,22*E*)-(1*S*,3*R*)-(1,3-Dihydroxy-24-oxo-24a-homo-9,10-secochola-5,7,10(19),22-tetraen-24a-yl)phosphonsäurebis(1-methylethyl)ester **13**

Vergleichssubstanz:

Calcitriol

| | KF | DR(HL 60) | DR (Ca) |
|---|---|---|---|
| **5** | 10 | 22 | >>100 |
| **9** | 10 | 58 | >300 |
| **13** | 6 | 32 | >300 |
| **Calcitriol** | 1 | 1 | 1 |

Die aufgeführten Verbindungen zeigen neben einer beträchtlichenAffinität zum Vitamin D-Rezeptor deutliche zelldifferenzierende Aktivität.

Die Induktion einer Hypercalcämie erfolgt dagegen durchweg erst bei sehr viel höheren Dosen als bei Calcitriol.

Bereits in nichthypercalcämischer Dosis wird der Aufbau von neuem Knochenmaterial induziert.

Durch die verminderte Eigenschaft, eine Hypercalcämie auszulösen sowie die hohe metabolische Stabilität, eignen sich die erfindungsgemäßen Substanzen in besonderer Weise zur Herstellung von Arzneimitteln für die Behandlung von Erkrankungen, die durch eine Hyperproliferation und fehlende Zelldifferenzierung gekennzeichnet sind. Dazu zählen zum Beispiel hyperproliferative Erkrankungen der Haut (Psoriasis, Pituriasis subia pilasis, Akne, Ichthyosis) und Pruritus sowie Tumorerkrankungen und Präkanzerosen (zum Beispiel Darmtumoren, Mammakarzinom, Lungentumoren, Prostatakarzinom, Leukämien, T-Zell-Lymphome, Melanome, Batazell Larzin, Squamous Carcinoma, aktinische Keratosen, Cervixdysplasien, metastasierende Tumore jeglicher Art).

Auch zur Behandlung und Prophylaxe von Erkrankungen, die durch eine Störung des Gleichgewichts des Immunsystems gekennzeichnet sind, eignen sich die erfindungsgemäßen Substanzen. Hierzu zählen Ekzeme und Erkrankungen des atopischen Formenkreises und entzündliche Erkrankungen (rheumatoide Arthritis, Atemwegserkrankungen z.B. Asthma) sowie Autoimmunerkrankungen wie zum Beispiel Multiple Sklerose, Diabetes mellitus Typ I, Myasthenia gravis, Lupus erythematodes, Sklerodermie, bullöse Hauterkrankungen (Pemphigus, Pemphigoid), weiterhin Abstoßungsreaktionen bei autologen, allogenen oder xenogenen Transplantaten sowie AIDS. Bei all diesen Erkrankungen können die neuen Verbindung der allgemeinen Formel **I** vorteilhaft mit anderen immunsuppressiv wirksamen Stoffen wie Cyclosporin A, FK 506, Rapamycin und Anti-CD 4-Antikörpern kombiniert werden.

Ebenso sind die Substanzen geeignet zur Therapie von sekundärem Hyperparathyreoidismus und renaler Osteodystrophie infolge der Eigenschaft von Calcitriolen, die Parathormonsynthese zu senken.

Aufgrund der Präsenz des Vitamin D-Rezeptor in den insulinproduzierenden Zellen der Bauchspeicheldrüse eignen sich die Substanzen durch Erhöhung der Insulinsekretion zur Therapie des Diabetes mellitus Typ II.

Weiterhin wurde überraschenderweise gefunden, daß durch topische Applikation der erfindungsgemäßen Verbindungen auf die Haut von Mäusen, Ratten und Meerschweinchen eine vermehrte Hautrötung und Zunahme der Epidermisdicke induziert werden kann. Die Zunahme der Hautrötung wird anhand der Erhöhung des mit einem Farbmeßgerät quantifizierbaren Rotwertes der Hautoberflache ermittelt. Der Rotwert ist nach dreimaliger Substanzapplikation (Dosis 0,003%) im Abstand von 24 Stunden typischerweise um das 1,5-fache erhöht. Die Zunahme der Epidermisdicke wird im histologischen Präparat quantifiziert. Sie ist typischerweise um das 2,5-fache erhöht. Die Anzahl der proliferierenden Epidermiszellen (Zellen in der S-Phase des Zellcyclus) wird durchflußcytometrisch ermittelt und ist typischerweise um den Faktor 6 erhöht.

Diese Eigenschaften der erfindungsgemäßen Derivate in der Vitamin D-Reihe laßt sie zum therapeutischen Einsatz bei atrophischer Haut, wie sie bei natürlicher Hautalterung infolge erhöhter Lichtexposition oder medikamentös induzierter Hautatrophie durch Behandlung mit Glucocorticoiden auftritt, geeignet erscheinen.

Darüber hinaus kann die Wundheilung durch topische Applikation mit den neuen Verbindungen beschleunigt werden.

In Zellpopulationen das Haarfollikels, die entscheidend zum Haarwachstum bzw. der Haarzyklusregulation beitragen, konnten Vitamin $D_3$-Rezeptorproteine nachgewiesen werden [Stumpf, W. E. et al., *Cell Tissue Res*. **238**, 489 (1984); Milde, P. et al., *J. Invest. Dermatol*. **97**, 230 (1991)]. Außerdem zeigen in vitro-Befunde an isolierten Haarfollikelkeratinozyten einen proliferationsinhibierenden und differenzierungsstimmulierenden Einfluß von 1,25-$(OH)_2D_3$.

Aus klinischen Beobachtungen ist bekannt, daß die Vitamin $D_3$-resistente Rachitis häufig mit einer Alopezie einhergeht, die sich im frühen Kindesalter ausprägt. Experimentelle Befunde zeigen, daß die Vitamin $D_3$-Bindungsstelle des VDR bei dieser Erkrankung mutiert, d. h. defekt ist [Kristjansson. K. et al., *J. Clin. Invest.* **92**, 12 (1993)] . Keratinozyten, die aus den Haarfollikeln dieser Patienten isoliert wurden, reagieren in vitro nicht auf die Zugabe von 1,25-$(OH)_2D_3$ [Arase, S. et al., *J. Dermatol. Science* **2**, 353 1991)].

Aus diesen Befunden läßt sich eine entscheidende Rolle von 1,25-$(OH)_2D_3$ auf die Regulation des Haarwuchstums ableiten.

Daher eignen sich diese Analoga besonders zur Herstellung von Arzneimitteln zur Behandlung von Erkrankungen, die mit einem gestörten Haarwachstum einhergehen (androgenetische Alopezie, Alopezia areata/totalis, chemotherapie-induzierte Alopezie), oder zur Unterstützung des physiologischen Haarwachstums ohne die Nebenwirkungen des Calcitriols (insbesondere Hypercalcämie) hervorzurufen.

Die senile und postmenopausale Osteoporose ist gekennzeichnet durch einen erhöhten Knochenumsatz mit einer insgesamt negativen Bilanz. Aufgrund des Knochenschwundes insbesondere von trabekulärem Knochen kommt es in verstärktem Maße zu Knochenbrüchen. Aufgrund der fördernden Wirkung von Calcitriol sowohl auf die Anzahl als auch die Syntheseleistung von knochenneubildenden Zellen (Osteoblasten) eignen sich die erfindungsgemäßen Substanzen zur Therapie und Prophylaxe der senilen und postmenopausalen Osteoporose (EP 0 634 173 A1), der steroidinduzierten Osteoporose sowie zur beschleunigten Einheilung von Gelenkplastiken ohne die Nebenwirkungen des Calcitriols (insbesondere Hypercalcämie) hervorzurufen. Für die Therapie der verschiedenen Formen der Osteoporose können sie vorteilhaft mit Estradiol oder anderen Abkömmlingen des Östrogens kombiniert werden.

Schließlich konnte gezeigt werden, daß Calcitriol die Synthese eines Wuchsstoffes für Nervenzellen (nerve growth factor) steigert [MS Saporito et al. *Brain Res.* **633**, 189 (1994)]. Daher eignen sich die erfindungsgemäßen Verbindungen auch zur Behandlung von degenerativen Erkrankungen des peripheren und zentralen Nervensystems, wie der Alzheimerschen Erkrankung und der amyotrophen Lateralsklerose.

Es wurde außerdem gefunden, daß bestimmte Verbindungen der allgemeinen Formel **I** in HL 60-Zellen überraschenderweise die Wirkung von Calcitriol antagonisieren (siehe auch WO 94/07853, WO 97/00242).

Solche Verbindungen können bei der Therapie von Hypercalcämien eingesetzt werden, wie zum Beispiel bei Hypervitaminose D oder Intoxikation mit Calcitriol und calcitriolartig wirksamen Substanzen, oder bei erhöhter extrarenaler Calcitriolsynthese bei granulomatösen Erkrankungen (Sarkoidose, Tuberkulose). Auch paraneoplastische Hypercalcämien (zum Beispiel bei osteolytischen Metastasen und Tumoren mit erhöhter Synthese von Parathormon-related peptide) sowie bei Hypercalcämie bei Hyperparathyreoidismus.

Weiterhin sind Calcitriolantagonisten zur Fertilitätskontrolle einzusetzen. Im Reproduktionstrakt weiblicher und männlicher Tiere wird der Vitamin D-Rezeptor exprimiert. Es ist bekannt, daß die weibliche und männliche Fertilität Vitamin D-defizienter Tiere herabgesetzt ist. Durch kurzfristige Substitution von Calcitriol kann die Reproduktionsleistung erhöht werden. Daher sind Calcitriolantagonisten in der Lage, die weibliche und männliche Fertilität zu beeinflussen.

Da Calcitriol unter bestimmten Bedingungen eine immunsuppressive Wirkung zeigt, sind Calcitriolrezeptorantagonisten auch als Immunstimulantien, z. B. bei Infektabwehrschwäche, AIDS einzusetzen.

Von Calcitriol ist bekannt, daß es das Haarwachstum modulieren kann. Calcitriolantagonisten können daher bei unerwünschtem Haarwachstum, z. B. beim Hirsutismus, therapeutische Verwendung finden.

Eine fördernde Rolle von Vitamin D auf die Bildung von arteriosklerotischen Plaques ist seit langem bekannt. In solchen Gefäßläsionen wird ein Calcitriol-reguliertes Protein, das Osteopontin, vermehrt gefunden, dem eine Rolle bei der Gefäßverkalkung zugeschrieben wird [R. Eisenstein et al. *Arch. Path.* **77**, 27 (1964), L.A. Fitzpatrick et al. *J. Clin. Invest.* **94**, 1597 (1994)]. Deshalb eignen sich Calcitriolantagonisten zur Therapie und Prophylaxe aller Erscheinungsformen der Arteriosklerose.

Schließlich eignen sich Calcitriolantagonisten infolge der Eigenschaft von Calcitriol, unspezifische Immunreaktionen von monocytären Zellen zu steigern, zur Therapie von entzündlichen Erkrankungen insbesondere chronischer Natur, wie rheumatoide Arthritis, Morbus Crohn, Colitis ulcerosa, und granulomatösen Erkrankungen wie Sarkoidose und anderen Fremdkörperreaktionen.

Für alle aufgezählten therapeutischen Anwendungen gilt, daß die erfindungsgemäßen Verbindungen in der Lage sind, in den genannten Krankheitsbildern eine therapeutische Wirkung zu erreichen, ohne die Nebenwirkungen des Calcitriols (insbesondere Hypercalcämie) hervorzurufen.

Die vorliegende Erfindung bezieht sich somit auf pharmazeutische Präparate, die mindestens eine Verbindung gemäß der allgemeinen Formel **I** zusammen mit einem pharmazeutisch verträglichen Träger enthalten.

Die Verbindungen können als Lösungen in pharmazeutisch verträglichen Solventien oder als Emulsionen, Suspensionen, oder Dispersionen in geeigneten pharmazeutischen Solventien oder Trägern oder als Pillen, Tabletten oder Kapseln, die in an sich bekannter Weise feste Trägerstoffe enthalten, formuliert werden. Für eine topische Anwendung werden die Verbindungen vorteilhafterweise als Cremes oder Salben oder in einer ähnlichen, zur topischen Anwendung geeigneten Arzneimittelform formuliert. Jede derartige Formulierung kann auch andere pharmazeutisch verträgliche und nichttoxische Hilfsstoffe enthalten, wie z.B. Stabilisatoren, Antioxidantien, Bindemittel, Farbstoffe,

Emulgatoren oder Geschmackskorrigentien. Die Verbindungen werden vorteilhafterweise durch Injektion, intravenöse Infusion in geeigneter steriler Lösungen, als Aerosol über Bronchien und Lunge oder als orale Dosierung über den Ernährungstrakt oder topisch in der Form von Cremes, Salben, Lotions oder geeigneter transdermaler Pflaster appliziert, wie in der EP-A 0 387 077 beschrieben ist.

| | |
|---|---|
| Die tägliche Dosis liegt bei | 0,1 µg/Patient/Tag - 1000 µg/Patient/Tag, |
| vorzugsweise | 1,0 µg/Patient/Tag - 500 µg/Patient/Tag. |

**Verfahren zur Herstellung der erfindungsgemäßen Verbindungen**

Die Herstellung der Vitamin D-Derivate der allgemeinen Formel **I** erfolgt erfindungsgemäß aus einer Verbindung der allgemeinen Formel **II**,

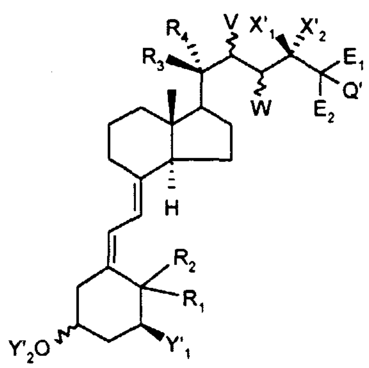

**II**

worin $Y'_1$ ein Wasserstoffatom, ein Halogenatom oder eine geschützte Hydroxylgruppe und $Y'_2$ eine Hydroxyschutzgruppe bedeuten.

$X'_1$, $X'_2$ und Q' unterscheiden sich von $X_1$, $X_2$ und Q dadurch, daß eventuell vorliegende Hydroxyl-, Amino- oder Ketogruppen in geschützter Form vorliegen können.

Bei den Schutzgruppen handelt es sich vorzugsweise um alkyl-, aryl- oder gemischt alkylarylsubstituierte Silylgruppen, z.B. die Trimethylsilyl- (TMS), Triethylsilyl-(TES), *tert.*-Butyldimethylsilyl- (TBDMS), *tert.*-Butyldiphenylsilyl- (TBDPS) oder Triisopropylsilylgruppen (TIPS) oder eine andere gängige Hydroxyschutzgruppe (Methoxymethyl-, Methoxyethoxymethyl-, Ethoxyethyl-, Tetrahydrofüranyl-Tetrahydropyranyl-Gruppen) für die Ketogruppen handelt es sich vorzugsweise um Ketale (1,3-Dioxolane, 1,3-Dioxane, Dialkoxyketale) (siehe T.W. Greene, P.G.M. Wuts "Protective Groups in Organic Synthesis", 2nd Edition, John Wiley & Sons, 1991).

Durch gleichzeitige oder sukzessive Abspaltung der Hydroxy- sowie Ketoschutzgruppen und gegebenenfalls durch partielle, sukzessive oder vollständige Veresterung der freien Hydroxylgruppen wird **II** in eine Verbindung der allgemeinen Formel **I** überführt.

Im Falle der Silylschutzgruppen oder der Trimethylsilylethoxymethylgruppe verwendet man zu deren Abspaltung Tetrabutylammoniumfluorid, Fluorwasserstoffsäure oder Fluorwasserstoffsäure/Pyridin oder saure Ionentauscher; im Falle von Ethergruppen (Methoxymethyl-, Methoxyethoxymethyl-, Ethoxyethyl-, Tetrahydropyranylether) und Ketalen werden diese unter katalytischer Einwirkung von Säure, beispielsweise p-Toluolsulfonsäure, Pyridinium-p-toluolsulfonat, Essigsäure, Salzsäure, Phosphorsäure oder einem sauren Ionenaustauscher abgespalten.

Die Veresterung der freien Hydroxygruppen kann nach gängigen Verfahren mit den entsprechenden Carbonsäurechloriden, -bromiden oder -anhydriden erfolgen.

Die Herstellung der Ausgangsverbindungen für die allgemeine Formel **II** geht je nach letztendlich gewünschtem Substitutionsmuster in 3-, 10- und 20-Position von verschiedenen Startverbindungen aus.

Für die Herstellung von Verbindungen der allgemeinen Formel **II**, worin $R_1$ und $R_2$ gemeinsam eine exocyclische Methylengruppe und $Y'_1$ ein Wasserstoffatom oder eine geschützte Hydroxylgruppe und $Y'_2$ eine Hydroxyschutzgruppe bedeuten sowie die natürliche Stereochemie in der 3-Position vorliegt, wird von dem bekannten Aldehyd **III** ausgegangen [M. Calverley Tetrahedron <u>**43**</u>, 4609(1987), WO 87/00834].

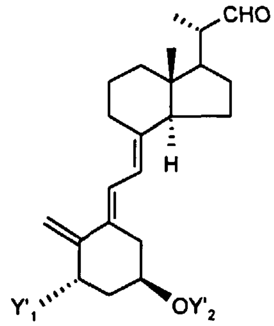

III

Andere Schutzgruppen für $Y'_1$ und $Y'_2$ als die in den Literaturstellen erwähnten lassen sich durch analoge Vorgehensweise unter Verwendung entsprechend modifizierter Silylchloride (z.B. *tert.* -Butyldiphenylsilylchlorid anstelle von *tert.* - Butyldimethylsilylchlorid) erhalten. Durch Verzicht auf die entsprechenden Stufen zur 1α-Hydroxylierung lassen sich Derivate vom Typ $Y'_1$ =H erhalten.

Die Verbindungen der allgemeinen Formel **III** werden nun analog bekannter Verfahren in Aldehyde der allgemeinen Formel **IV** überführt [EP 647 219, WO 94/07853, M.J. Calverley, L. Binderup *Bioorg. Med. Chem. Lett.* **3**, 1845-1848 (1993)].

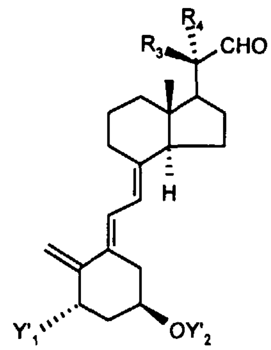

IV

Für $R_3$ und $R_4$ gelten die schon eingangs erwähnten Definitionen.

Zur Etablierung des natürlichen Vitamin D-Triensystems wird eine photochemische Isomerisierung von Verbindungen der allgemeinen Formel **IV** vorgenommen. Bestrahlung mit ultraviolettem Licht erfolgt in Gegenwart eines sogenannten Triplettsensibilisators. Im Rahmen der vorliegenden Erfindung wird dafür Anthracen verwendet. Durch Spaltung der π-Bindung der 5,6-Doppelbindung, Rotation des A-Ringes um 180° um die 5,6-Einfachbindung und Reetablierung der 5,6-Doppelbindung wird die Stereoisomerie an der 5,6-Doppelbindung umgekehrt, wobei Verbindungen der allgemeinen Formel **V** anfallen,

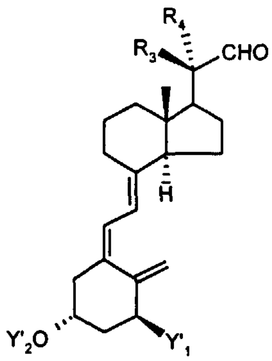

V

Grundsätzlich ist diese Isomerisierungsreaktion auch auf einer späteren Stufe möglich. Exemplarisch werden im Folgenden die Umsetzungen des Aldehydes **VI** mit natürlicher Konfiguration an C-20 beschrieben.

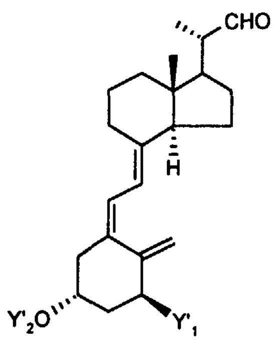

VI

Prinzipiell sind die folgenden Umsetzungen aber auch mit den genannten Substitutionsmustern an C-20 möglich.

Durch Umsetzung des Aldehydes der allgemeinen Formel **VI** mit Trialkylphosphonoacetaten der allgemeinen Formel **VII** oder Amiden der allgemeinen Formel **VIII**,

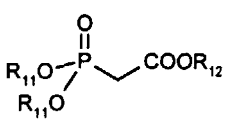

VII

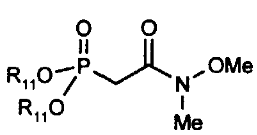

VIII

wobei $R_{11}$ und $R_{12}$ aliphatische oder aromatische Reste mit 1 bis 12 C-Atomen bedeuten, werden Ester der allgemeinen Formel **IX** oder Amide der allgemeinen Formel **X** erzeugt.

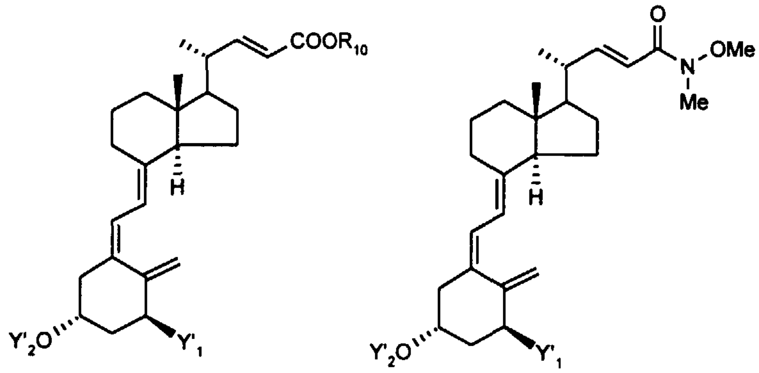

IX X

Die Verbindungen der allgemeinen Formel **IX** sowie **X** können nun mit durch eine Base wie zum Beispiel *n*-Butyllithium, *t*-Butyllithium, Lithiumdiisopropylamid, Natriumhydrid, Kaliumhydrid, Natriumhydroxid, Kaliumhydroxid Natriumalkoholate, Kaliumalkoholate usw. deprotonierten Phosphorverbindungen der allgemeinen Formel **XI** umgesetzt werden.

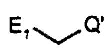

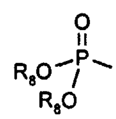

**XI** **XII**

$E_1$ und Q' sind bereits zuvor definiert worden. Einen Spezialfall stellt exemplarisch der Methylphosphonsäureester der allgemeinen Formel **XII** dar.

Somit erhält man Verbindungen der allgemeinen Formel **II**, für die gilt, daß $X'_1$ und $X'_2$ gemeinsam eine Ketogruppe bilden. Diese Verbindungen können wie zuvor beschrieben in Verbindungen der allgemeinen Formel **I** überführt werden oder mit einem Redüktionsmittel wie zum Beispiel Natriumborhydrid, Natriumborhydrid/Certrichlorid (Heptahydrat), Diisobutylaluminiumhydrid, Lithiumborhydrid usw. in Verbindungen der allgemeinen Formel **II** überführt werden, für die gilt $X'_1$ ist eine Hydroxylgruppe und $X'_2$ ist ein Wasserstoffatom oder $X'_1$ ist ein Wasserstoffatom und $X'_2$ ist eine Hydroxylgruppe. Die Überführung in die Verbindungen der allgemeinen Formel **I** gelingt wie vorstehend beschrieben.

Alternativ können die letzteren Verbindungen der allgemeinen Formel **II** auch durch Umsetzung der Phosphorverbindung der allgemeinen Formel **XI** mit einem Aldehyd der allgemeinen Formel **XIII**, welcher durch Reduktion des Esters der allgemeinen Formel **IX** oder des Amids der allgemeinen Formel **X** mit einem Redüktionsmittel wie z.B. Diisobutylaluminiumhydrid, Lithiumaluminiumhydrid usw. erhalten werden kann, synthetisiert werden.

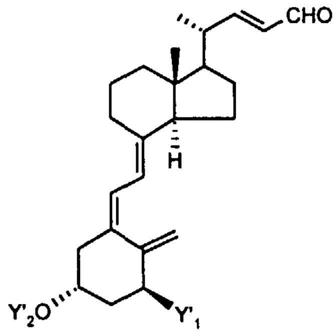

XIII

Zur Synthese von Verbindungen der allgemeinen Formel **II**, für die gilt, daß $X'_1$ und $X'_2$ Wasserstoffatome sind, verwendet man Derivate des Alkohols der allgemeinen Formel **XIV**, die mit der allgemeinen Formel **XV** bezeichnet werden können.

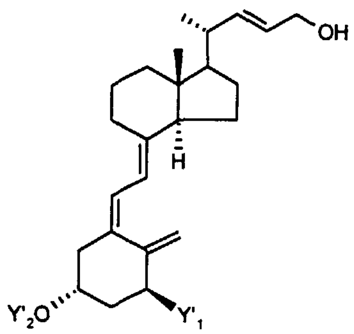

XIV

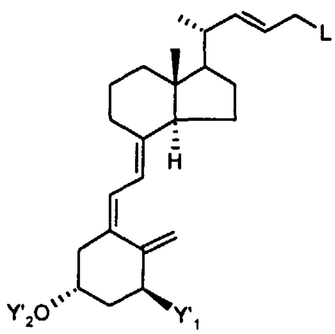

XV

Der Alkohol der allgemeinen Formel **XIV** kann aus dem Aldehyd der allgemeinen Formel **XIII**, dem Ester der allgemeinen Formel **IX** oder Amid der allgemeinen Formel **X** mit einem Reduktionsmittel wie zum Beispiel Diisobutylaluminiumhydrid, Lithiumaluminiumhydrid, Natriumborhydrid` Lithiumborhydrid usw. erhalten werden. Die Hydroxylgruppe muß dann unter den üblichen Bedingungen in eine Fluchtgruppe umgewandelt werden wie zum Beispiel ein Methansulfonsäureester, p-Toluolsulfonsäureester oder ein Halogenatom (Fluor, Chlor, Brom). Eine Isolierung dieser Verbindung ist nicht unbedingt notwendig. Nun kann die Reaktion mit einer durch eine Base wie zum Beispiel *n*-Butyllithium, *t*-Butyllithium, Lithiumdiisopropylamid, Natriumhydrid, Kaliumhydrid, Natriumhydroxid, Kaliumhydroxid Natriumalkoholate, Kaliumalkoholate usw. deprotonierten Phosphorverbindung der allgemeinen Formel **XVI**

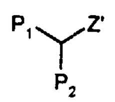

XVI

zu Verbindungen der allgemeinen Formel **II** erfolgen, die wie beschrieben in eine Verbindung der allgemeinen Formel **I** umgewandelt wird. $E_1$, $E_2$ und Q' sind bereits zuvor definiert worden.
Die Verbindung der allgemeinen Formel **XVI** kann auch unter Palladium-Katalyse mit Verbindungen der allgemeinen Formel **XIV** oder der allgemeinen Formel **XV** für die gilt, daß L Acetat, Carbonat oder ähnliche Gruppen bedeutet [siehe D.E. Bergbreiter et al. *J Chem. Soc., Chem. Comm.* 883-884 (1989), J. Tsuji "Organic Synthesis with Palladium Compounds", Springer Verlag, Berlin, 1980], wobei Verbindungen der allgemeinen Formel **II** der vorstehenden Bedeutung

gebildet werden und wie beschrieben in Verbindungen der allgemeinen Formel **I** umgewandelt werden können.

Für den Fall, daß in der allgemeinen Formel **II** $X'_1$ und $X'_2$ zusammen eine Ketogruppe bilden und $E_1$ und $E_2$ gleichzeitig phosphorhaltige Gruppen darstellen, kann die Synthese auch ausgehend vom Säurederivat der allgemeinen Formel **XVII** erfolgen,

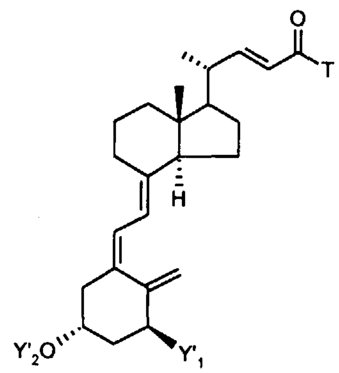

**XVII**

wobei T ein Halogenid, eine Cyanidgruppe oder eine andere aktivierende Gruppe (z.B. Anhydrid) bedeutet. Die Darstellung der Verbindung der allgemeinen Formel **XVII** gelingt nach Hydrolyse der Estergruppe der allgemeinen Formel **IX** und Funktionalisierung unter den üblichen Bedingungen.
Nun kann die Reaktion mit einer durch eine Base wie zum Beispiel *n*-Butyllithium, *t*-Butyllithium, Lithiumdiisopropylamid, Natriumhydrid, Kaliumhydrid, Natriumhydroxid, Kaliumhydroxid Natriumalkoholate, Kaliumalkoholate usw. deprotonierten Phosphorverbindung der allgemeinen Formel **XVI** zu Verbindungen der allgemeinen Formel **II** erfolgen, die wie beschrieben in Verbindungen der allgemeinen Formel **I** umgewandelt werden.

Einen Sonderfall stellt die Reaktion des Aldehydes der allgemeinen Formel **XIII** mit einer Verbindung der allgemeinen Formel **XVI** dar, für die gilt, daß $E_1$ und $E_2$ gleich sind. Bei dieser Reaktion wird unter Eliminierung einer phosphorhaltigen Gruppe eine Verbindung der allgemeinen Formel **XVIII** generiert,

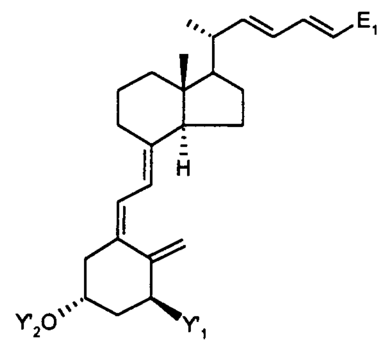

**XVIII**

die als Spezialfall der allgemeinen Formel **II** anzusehen ist, für den gilt $X'_1$ und $E_2$ bilden zusammen eine *E*-Doppelbindung und $X'_2$ und Q' sind Wasserstoffatome. Die weitere Umsetzung zu Verbindungen der allgemeinen Formel **I** kann wie beschrieben erfolgen.

Für den Fall, daß in der allgemeinen Formel **II** $X'_1$ und $E_2$ zusammen eine Doppelbindung bilden und $X'_2$ eine Gruppe -OZ bedeutet, wird eine Verbindung der allgemeinen Formel **II**, für die gilt, daß $X'_1$ und $X'_2$ zusammen eine Ketogruppe bilden, $E_1$ die beschriebene Definition hat und $E_2$ ein Wasserstoffatom bedeutet, mit einer Base wie zum Beispiel *n*-Butyllithium, *t*-Butyllithium, Lithiumdiisopropylamid, Natriumhydrid, Kaliumhydrid, Natriumhydroxid, Kaliumhydroxid Natriumalkoholaten, Kaliumalkoholaten deprotoniert und anschließend mit einer Verbindung der allgemeinen Formel **XIX** umgesetzt,

L-Z

**XIX**

wobei L eine Fluchtgruppe vorstehender Bedeutung ist und Z zuvor definiert wurde. Die weitere Umsetzung zu Verbindungen der allgemeinen Formel **I** kann wie beschrieben erfolgen.

Für den Fall, daß $X'_1$ und $X'_2$ in der allgemeinen Formel **II** zusammen eine Carbonylgruppe bedeuten, $E_1$ und $E_2$ die vorstehenden Bedeutungen haben und Q' nicht Wasserstoff ist, wird eine Verbindung der allgemeinen Formel **II**, für die gilt daß $X'_1$ und $X'_2$ eine Carbonylgruppe bedeuten und $E_1$ und $E_2$ die vorstehenden Bedeutungen haben und Q' ein Wasserstoff ist, mit einer Base wie zum Beispiel *n*-Butyllithium, *t*-Butyllithium, Lithiumdiisopropylamid, Natriumhydrid, Kaliumhydrid, Natriumhydroxid, Kaliumhydroxid Natriumalkoholaten, Kaliumalkoholaten deprotoniert und anschließend mit einer Verbindung der allgemeinen Formel **XX** umgesetzt,

L-Q'

**XX**

wobei L eine Fluchtgruppe vorstehender Bedeutung ist und Q zuvor definiert wurde. Alternativ können auch palladiumkatalysierte Verknüpfungsreaktionen zur Anwendung kommen. In diesem Fall muß L eine Hydroxygruppe, ein Acetat, Carbonat oder eine vergleichbare Gruppe sein [siehe D.E. Bergbreiter et al. *J Chem. Soc. Chem. Comm.* 883-884 (1989), J. Tsuji "Organic Synthesis with Palladium Compounds", Springer Verlag, Berlin, 1980]. Die weitere Umsetzung zu Verbindungen der allgemeinen Formel **I** kann wie beschrieben erfolgen.

Verbindungen der allgemeinen Formel **II**, für die gilt, daß V und W Wasserstoffatome bedeuten können durch Reduktion der 22,23-Doppelbindung am Ester **IX**, Amid **X** oder Aldehyd **XIII** unter den gängigen Reduktionsbedingungen [Natriumborhydrid/Pyridin; Magnesium in Methanol; Kupferhydrid, generiert in situ, M.F. Semmelhack et al. *J. Org. Chem.* **40**, 3619-3621(1975); Samariumiodid, H. Alper *Tetrahedron Lett.* **33**, 5007-5008 (1992); Natriumdithionit, K.G. Akamanchi et al. *Synth. Comm.* **22**, 1655-1660 (1992) usw.] und weiteren Reaktionen (wie beschrieben) hergestellt werden. Daneben können durch zusätzliche Reduktion der Alkohol der allgemeinen Formel **XXI** sowie Derivate der allgemeinen Formel **XXII** synthetisiert und wie beschrieben umgesetzt werden.

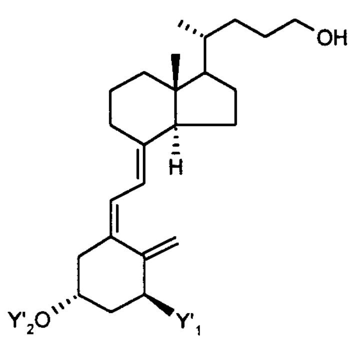

**XXI**

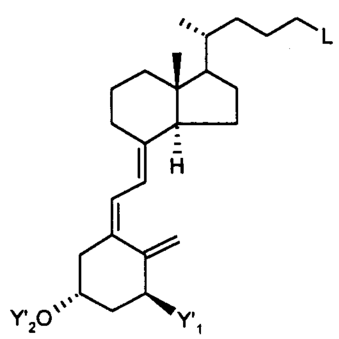

**XXII**

Zur Synthese von Verbindungen der allgemeinen Formel **II** für die $R_1$ und $R_2$ Wasserstoffatome sind, muß ein konvergenter Syntheseweg beschritten werden, bei dem CD- und A-Ring-Fragmente separat aufgebaut werden. Zur Synthese der CD-Fragmente wird der literaturbekannte Aldehyd **XXIII** [H.H. Inhoffen et al. *Chem. Ber.* **92**, 781-791(1958); H.H. Inhoffen et al. *Chem. Ber.* **92**, 1772-1788 (1959); W.G. Dauben et al. *Tetrahedron Lett.* **30**, 677-680 (1989)] verwendet,

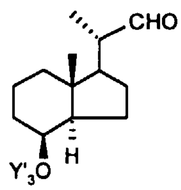

**XXIII**

worin $Y'_3$ eine acyl-, alkyl- oder arylsubstituierte Silyl- oder eine Tetrahydropyranyl-, Tetrahydrofuranyl-, Methoxymethyl-, Ethoxyethyl-, eine Acylgruppe (z.B. Acetyl-, Benzoylgruppe) oder eine andere Hydroxyschutzgruppe bedeutet (siehe T.W. Greene, P.G.M. Wuts *Protective Groups in Organic Synthesis*, $2^{nd}$ Edition, John Wiley and Sons, Inc. 1991).

Nach den bekannten Verfahren können hier die schon beschriebenen Modifikationen an C-20 eingeführt werden (WO 94/07853), wobei eine Verbindung der allgemeinen Formel **XXIV** anfallt.

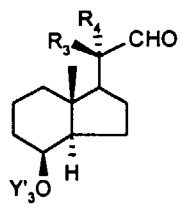

**XXIV**

Die Einführung der Seitenketten erfolgt hier in Analogie zum Fall des Vitamin D-Aldehydes **VI**, wobei man Verbindungen der allgemeinen Formel **XXV** erhält.

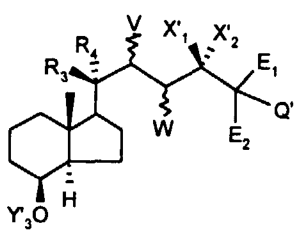

**XXV**

Die Variablen wurden vorher bereits definiert.

Bei der Wahl geeigneter Schutzgruppen (z.B. $Y'_3$ =Triethylsilyl) wird $Y'_3$ selektiv gespalten (z.B. mit Tetrabutylammoniumfluorid), wobei die Verbindung der allgemeinen Formel **XXVI** anfällt.

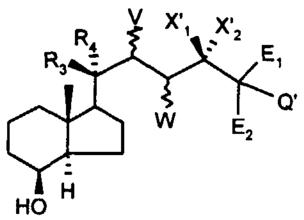

XXVI

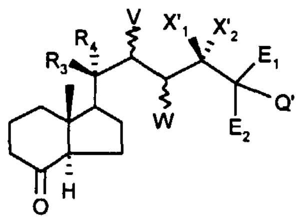

XXVII

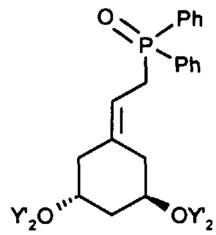

XXVIII

Oxidation nach bekannter Methode (z.B. Pyridiniumchlorochromat, Pyridiniumdichromat, Swern-Bedingungen usw.) ergeben eine Verbindung der allgemeinen Formel **XXVII**, die durch Reaktion mit dem durch eine Base (z.B. Lithiumdiisopropylamid, *n*-Butyllithium) erzeugten Anion des literaturbekannten Phosphinoxides der allgemeinen Formel **XXVIII** [H.F. DeLuca ei al. *Tetrahedron Lett.* __32__, 7663-7666 (1991)], worin $Y'_2$ die schon beschriebene Bedeutung hat, in entsprechende Verbindungen der allgemeinen Formel **II** für die gilt: $Y'_1$=$OY'_2$ überführt wird. Die weitere Umsetzung in die Verbindung der allgemeinen Formel **I** erfolgt wie schon beschrieben.

Für die Synthese von Verbindungen der allgemeinen Formel **II** für die die Gruppe $Y_2O$ in der 3α-Position steht, muß auf einer frühen Stufe eine Epimerisierung vorgenommen werden. Exemplarisch ist dies für die Verbindung **XXIX** gezeigt. Der Prozeß ist aber grundsätzlich auch mit anderen Seitenkettensubstrukturen möglich.

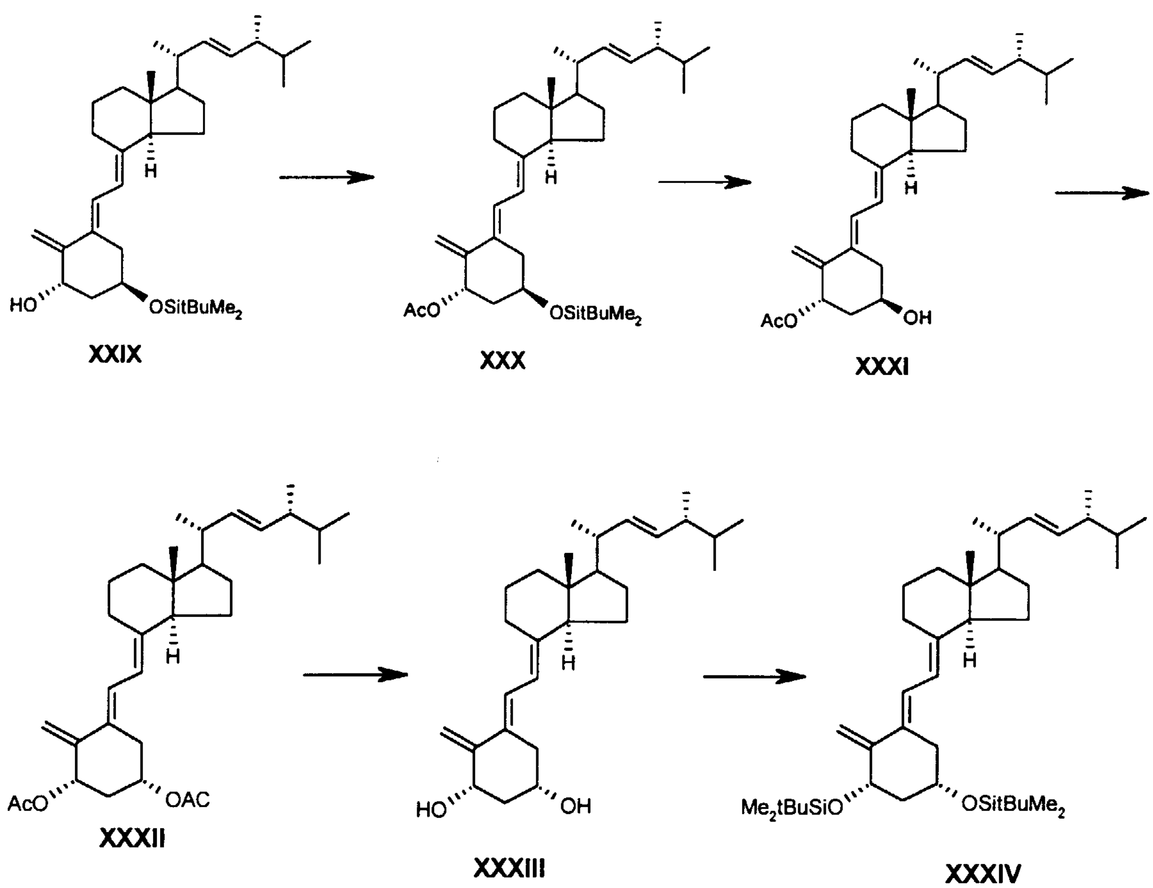

XXIX XXX XXXI

XXXII XXXIII XXXIV

Die im gezeigte Schutzgruppenstrategie stellt nur ein Beispiel für die Erzeugung des 3-epi-Derivates dar. Grundsätzlich sind diverse andere Strategien möglich. Durch Acetylierung der freien Hydroxylgruppe und Abspaltung der Silyleinheit gelangt man hier zum Alkohol **XXXI**, der nun nach bekannten Methoden invertiert werden kann (Tosylierung oder Mesylierung und nukleophile Substitution; Mitsonobu-Inversion usw.). Wechsel der Schutzgruppen führt zurück in die literaturbekannte Sequenz [M. *Calverley Tetrahedron* **43**, 4909 (1987)]. Die Verbindung **XXXIV** kann genau wie die entsprechende Verbindung der Normalreihe behandelt werden und kann alle für diese beschrieben Produkte - in der 3-epi-Serie - liefern.

Die nachstehenden Beispiele dienen der näheren Erläuterung der Erfindung.

**Beispiel 1**

(5*Z*,7*E*,22*E*)-(1*S*,3*R*)-(1,3-Dihydroxy-24-oxo-24a-homo-9,10-secochola-5,7,10(19),22-tetraen-24a-yl)phosphonsäurebismethylester **5**

a) Man löst 7,5 g (5*E*,7*E*)-(1*S*,3*R*)-1,3-Bis[[dimethyl(1,1-dimethylethyl)silyl]oxy]-9,10-secopregna-5,7,10(19)-trien-20-carbaldehyd **1** [M.J. Calverley *Tetrahedron* **43**, 4609-4619 (1987)] in 200 ml Toluol, fügt 2 g Anthracen und 0,5 ml Triethylamin hinzu und bestrählt unter Stickstoffdurchleitung in einer Pyrex-Apparatur mit einer Quecksilberhochdrucklampe für 30 min. Anschließend filtriert man, engt ein und chromatographiert den Rückstand an Silicagel mit Essigester/Hexan, wobei man 7,1 g (5*Z*,7*E*)-(1*S*,3*R*)-1,3-Bis[[dimethyl(1,1-dimethylethyl)silyl]oxy]-9,10-secopregna-5,7,10(19)-trien-20-carbaldehyd **2** als farblosen Schaum erhält.

$^1$H-NMR (300 MHz, $CDCl_3$): δ=0,05 ppm (s, 12H); 0,55 (s, 3H); 0,88 (s, 18H); 1,11 (d, 3H); 2,37 (m, 1H); 4,18 (m, 1H); 4,37 (m, 1H); 4,84 (s, 1H); 5,17 (s, 1H); 6,00 (d, 1H); 6,22 (d, 1H); 9,58 (d, 1H)

b) Man legt 455 mg Natriumhydrid (60 % Suspension in Paraffinöl) in 40 ml Tetrahydrofuran (THF) unter Stickstoff vor und tropft bei 0°C 2,69 g Trimethylphosphonoacetat in 5 ml THF zu. Nach 30 Minuten werden 1,8 g des Aldehydes **2** in 20 ml THF zugetropft. Man rührt 1 h bei Raumtemperatur nach und quencht anschließend mit Natriumchlorid-Lösung. Es wird mit Essigester extrahiert, die organische Phase mit Natriumchlorid-Lösung gewaschen, über Natriumsulfat getrocknet und eingeengt. Den Rückstand reinigt man durch Chromatographie an Silicagel mit Essigester/Hexan, wobei 1,4 g (5*Z*,7*E*,22*E*)-(1*S*,3*R*)-1,3-Bis[[dimethyl(1,1-dimethylethyl)silyl]oxy]-9,10-secochola-5,7,10(19),22-tetraen-24-säuremethylester **3** als farbloser Schaum erhalten werden.

$^1$H-NMR (300 MHz, $CDCl_3$): δ= 0,05 ppm (s, 12 H); 0,57 (s, 3H); 0,90 (s, 18H); 1,09 (d, 3H); 3,72 (s, 3H); 4,19 (m, 1H); 4,38 (m, 1H); 4,87 (s, 1H); 5,19 (s, 1H); 5,77 (d, 1H); 6,02 (d, 1H); 6,23 (d, 1H); 6,87 (dd, 1H)

c) Man legt 90 mg Methanphosphonsäuredimethylester in 5 ml THF unter Stickstoff vor und kühlt auf -78°C. Es werden nun 0,48 ml *n*-Butyllithium-Lösung (1.6 M in Hexan) zugetropft und es wird 30 min bei dieser Temperatur gerührt. Anschließend tropft man 430 mg des Esters **3** in 1 ml THF zu und rührt weitere 30 min bei -78°C nach. Es wird mit Natriumchlorid-Lösung gequencht, mit Essigester extrahiert, die organische Phase mit Natriumchlorid-Lösung gewaschen, über Natriumsulfat getrocknet und eingeengt. Der Rückstand wird durch Chromatographie an Silicagel mit Essigester/Hexan gereinigt, wobei man 376 mg (5*Z*,7*E*,22*E*)-(1*S*,3*R*)-[1,3-Bis[[dimethyl(1,1-dimethylethyl)silyl]oxy]-24-oxo-24a-homo-9,10-secochola-5,7,10(19),22-tetraen-24a-yl]phosphon-säuredimethylester **4** als farblosen Schaum erhält.

$^1$H-NMR (300 MHz, $CD_2Cl_2$): δ= 0,05 ppm (s, 12 H); 0,54 (s, 3H); 0,86 (s, 18H); 1,10 (d, 3H); 3,16 (d, 2H); 3,71 (s, 3H); 3,73 (s, 3H); 4,16 (m, 1H); 4,36 (m, 1H); 4,82 (s, 1H); 5,16 (s, 1H); 6,01 (d, 1H); 6,07 (d, 1H); 6,23 (d, 1H); 6,72 (dd, 1H)

d) Man löst 35 mg des Phosphonsäureesters **4** in einem Gemisch aus 1 ml Dichlormethan und 9 ml Methanol und rührt unter Stickstoff mit 350 mg aktiviertem saurem Dowex-Ionentauscher über Nacht bei Raumtemperatur. Man filtriert, wäscht das Filtrat mit Natriumhydrogencarbonat-Lösung und Natriumchlorid-Lösung, trocknet über Natriumsulfat und entfernt das Lösungsmittel. Der Rückstand wird durch Chromatographie an Silicagel mit Essigester/Hexan gereinigt, wobei man 16 mg der Titelverbindung **5** als farblosen Schaum erhält.

$^1$H-NMR (300 MHz, $CD_2Cl_2$): δ= 0,57 ppm (s, 3 H); 1,10 (d, 3H); 3,18 (d, 2H); 3,68 (s, 3H); 3,74 (s, 3H); 4,17 (m, 1H); 4,37 (m, 1H); 4,96 (s, 1H); 5,28 (s, 1H); 6,00 (d, 1H); 6,09 (d, 1H); 6,35 (d, 1H); 6,77 (dd, 1H)

**Beispiel 2**

(5*Z*,7*E*,22*E*)-(1*S*,3*R*,24*S*)-(1,3,24-Trihydroxy-24a-homo-9,10-secochola-5,7,10(19),22-tetraen-24a-yl)phosphonsäuredimethylester **7a** und (5*Z*,7*E*,22*E*)-(1*S*,3*R*,24*R*)-(1,3,24-Trihydroxy-24a-homo-9,10-secochola-5,7,10(19),22-tetraen-24a-yl)phosphonsäuredimethylester **7b**

a) Man löst 80 mg des Ketons **4** in 2 ml Dichlormethan und fügt unter Stickstoff 8 ml Ethanol hinzu. Bei 0°C gibt man nacheinander 52 mg Certrichlorid (Heptahydrat) und 6 mg Natriumborhydrid zu und rührt 15 min nach. Anschließend wird Natriumchlorid-Lösung zugegeben, mit Essigester extrahiert, die organische Phase mit Natriumchlorid-Lösung gewaschen und über Natriumsulfat getrocknet. Nach Einengen chromatographiert man den Rückstand an Silicagel mit Essigester/Hexan und erhält 61 mg (5*Z*,7*E*,22*E*)-(1*S*,3*R*)-[1,3-Bis[[dimethyl(1,1-dimethylethyl)silyl]oxy]-24-hydroxy-24a-homo-9,10-secochola-5,7,10(19),22-tetraen-24a-yl]phosphonsäuredimethylester **6** als farblosen Schaum (1:1-Gemisch der Diastereomeren bzgl. C-24).

$^1$H-NMR (300 MHz, $CD_2Cl_2$): δ= 0,05 ppm (s, 12 H); 0,53 (s, 3H); 0,86 (s, 18H); 1,01/1,02 (jeweils d, 3H); 3,17/3,23 (jeweils d, OH); 3,68 (d, 3H); 3,72 (d, 3H); 4,16 (m, 1H); 4,35 (m, 1H); 4,36 (m, 1H); 4,82 (s, 1H); 5,16 (s, 1H); 5,37/5,38 (jeweils dd, 1H); 5,49/5,51 (jeweils dd, 1H); 6,01 (d, 1H); 6,23 (d, 1H)

b) Man behandelt 61 mg des Diastereomerengemisches **6** analog 1d) und trennt die Epimeren schließlich über HPLC, wobei man nacheinander 12 mg der Titelverbindung **7a** und 11 mg der Titelverbindung **7b** jeweils als farblosen Schaum erhält.

$^1$H-NMR (300 MHz, $CD_2Cl_2$): **7a**: δ= 0,58 ppm (s, 3 H); 1,02 (d, 3H); 3,21 (sbr, OH); 3,71 (d, 3H); 3,77 (d, 3H); 4,18 (m, 1H); 4,38 (m, 1H); 4,41 (m, 1H); 4,97 (s, 1H); 5,30 (s, 1H); 5,41 (dd, 1H); 5,58 (dd, 1H); 6,01 (d, 1H); 6,35 (d, 1H)
**7b**: δ= 0,58 ppm (s, 3 H); 1,03 (d, 3H); 3,28 (sbr, OH); 3,71 (d, 3H); 3,77 (d, 3H); 4,18 (m, 1H); 4,38 (m, 1H); 4,42 (m, 1H); 4,97 (s, 1H); 5,30 (s, 1H); 5,39 (dd, 1H); 5,55 (dd, 1H); 6,01 (d, 1H); 6,35 (d, 1H)

**Beispiel 3**

(5*Z*,7*E*,22*E*)-(1*S*,3*R*)-(1,3-Dihydroxy-24-oxo-24a-homo-9,10-secochola-5,7,10(19),22-tetraen-24a-yl)phosphonsäurediethylester **9**

a) Man setzt 867 mg Methanphosphonsäurediethylester mit 1,2 g des Esters **3** analog 1c) um und erhält 1,05 g (5*Z*,7*E*,22*E*)-(1*S*,3*R*)-[1,3-Bis[[dimethyl(1,1-dimethylethyl)silyl]oxy]-24-oxo-24a-homo-9,10-secochola-5,7,10(19),22-tetraen-24a-yl]phosphonsäurediethylester **8** als farblosen Schaum.

$^1$H-NMR (300 MHz, $CDCl_3$): δ= 0,06 ppm (s, 12 H); 0,57 (s, 3H); 0,89 (s, 18H); 1,11 (d, 3H); 1,33 (t, 6H); 3,19 (d, 2H); 4,13 (q, 2H); 4,16 (q, 2H); 4,17 (m, 1H); 4,37 (m, 1H); 4,84 (s, 1H); 5,19 (s, 1H); 6,01 (d, 1H); 6,18 (d, 1H); 6,23 (d, 1H); 6,81 (dd, 1H)

b) Man behandelt 80 mg des Phosphonsäureesters **8** analog 1d) und erhält 47 der Titelverbindung **9** als farblosen Schaum.

$^1$H-NMR (300 MHz, $CD_2Cl_2$): δ=0,56 ppm (s, 3H); 1,08 (d, 3H); 1,27 (t, 6H); 3,16 (d, 2H); 4,05 (q, 2H); 4,07 (q, 2H); 4,16 (m, 1H); 4,37 (m, 1H); 4,95 (s, 1H); 5,29 (s, 1H); 6,00 (d, 1H); 6,09 (d, 1H); 6,35 (d, 1H); 6,76 (dd, 1H)

**Beispiel 4**

(5*Z*,7*E*,22*E*)-(1*S*,3*R*,24*S*)-(1,3,24-Trihydroxy-24a-homo-9,10-secochola-5,7,10(19),22-tetraen-24a-yl)phosphonsäurediethylester **11a** und (5*Z*,7*E*,22*E*)-(1*S*,3*R*,24*R*)-(1,3,24-Trihydroxy-24a-homo-9,10-secochola-5,7,10(19),22-tetraen-24a-yl)phosphonsäurediethylester **11b**

Man behandelt 300 mg des Phosphonsäureesters **8** analog 2a) und erhält 190 mg (5*Z*,7*E*,22*E*)-(1*S*,3*R*)-[1,3-Bis[[dimethyl(1,1-dimethylethyl)silyl]oxy]-24-hydroxy-24a-homo-9,10-secochola-5,7,10(19),22-tetraen-24a-yl]phosphonsäurediethylester **10** als farblosen Schaum (1:1-Gemisch der Diastereomeren bzgl. C-24).

$^1$H-NMR (300 MHz, $CD_2Cl_2$): δ= 0,06 ppm (s, 12 H); 0,52 (s, 3H); 0,86 (s, 18H); 1,01/1,02 (jeweils d, 3H); 1,30 (t, 6H); 4,05 (q, 2H); 4,08 (q, 2H); 4,16 (m, 1H); 4,35 (m, 1H); 4,37 (m, 1H); 4,83 (s, 1H); 5,16 (s, 1H); 5,38/5,39 (jeweils dd, 1H); 5,50/5,51 (jeweils dd, 1H); 6,01 (d, 1H); 6,23 (d, 1H)

b) Man behandelt 170 mg des Diastereomerengemisches **10** analog 1d) und trennt die Epimeren schließlich über HPLC, wobei man nacheinander 36 mg der Titelverbindung **11a** und 28 mg der Titelverbindung **11b** jeweils als farblosen Schaum erhält.

$^1$H-NMR (300 MHz, $CD_2Cl_2$): **11a**: δ= 0,55 ppm (s, 3 H); 1,02 (d, 3H); 1,31 (t, 6H); 4,07 (q, 2H); 4,09 (q, 2H); 4,17 (m, 1H); 4,37 (m, 1H); 4,40 (m, 1H); 4,96 (s, 1H); 5,29 (s, 1H); 5,40 (dd, 1H); 5,57 (dd, 1H); 6,01 (d, 1H); 6,36 (d, 1H)
**11b**: δ= 0,55 ppm (s, 3 H); 1,02 (d, 3H); 1,30 (t, 6H); 4,07 (q, 2H); 4,09 (q, 2H); 4,17 (m, 1H); 4,38 (m, 1H); 4,41 (m, 1H); 4,96 (s, 1H); 5,29 (s, 1H); 5,39 (dd, 1H); 5,54 (dd, 1H); 6,01 (d, 1H); 6,36 (d, 1H)

**Beispiel 5**

(5*Z*,7*E*,22*E*)-(1*S*,3*R*)-(1,3-Dihydroxy-24-oxo-24a-homo-9,10-secochola-5,7,10(19),22-tetraen-24a-yl)phosphonsäurebis(1-methylethyl)ester **13**

a) Man setzt 240 mg Methanphosphonsäurediisopropylester mit 500 mg des Esters **3** analog 1c) um und erhält 476 mg (5*Z*,7*E*,22*E*)-(1*S*,3*R*)-[1,3-Bis[[dimethyl(1,1-dimethylethyl)silyl]oxy]-24-oxo-24a-homo-9,10-secochola-5,7,10(19),22-tetraen-24a-yl]phosphonsäurebis(1-methylethyl)ester **12** als farblosen Schaum.

$^1$H-NMR (300 MHz, $CDCl_3$): δ=0,07 ppm (s, 12H); 0,58 (s, 3H); 0,89 (s, 18H); 1,12 (d, 3H); 1,33 (d, 12H); 3,16 (d, 2H); 4,18 (m, 1H); 4,38 (m, 1H); 4,72 (hept, 2H); 4,86 (s, 1H); 5,18(s, 1H); 6,01 (d, 1H); 6,19 (d, 1H); 6,23 (d, 1H); 6,80 (dd, 1H)

b) Man behandelt 70 mg des Phosphonsäureesters **12** analog 1d) und erhält 34 mg der Titelverbindung **13** als farblosen Schaum.

$^1$H-NMR (300 MHz, $CD_2Cl_2$): δ=0,58 ppm (s, 3H); 1,10 (d, 3H); 1,30 (d, 12H); 3,10 (d, 2H); 4,18 (m, 1H); 4,38 (m, 1H); 4,68 (hept, 2H); 4,96 (s, 1H); 5,29 (s, 1H); 6,01 (d, 1H); 6,12 (d, 1H); 6,36 (d, 1H); 6,76 (dd, 1H)

**Beispiel 6**

(5*Z*,7*E*,22*E*)-(1*S*,3*R*,24*S*)-(1,3,24-Trihydroxy-24a-homo-9,10-secochola-5,7,10(19),22-tetraen-24a-yl)phosphonsäurebis(1-methylethyl)ester **15a** und (5*Z*,7*E*,22*E*)-(1*S*,3*R*,24*R*)-(1,3,24-Trihydroxy-24a-homo-9,10-secochola-5,7,10(19),22-tetraen-24a-yl)phosphonsäurebis(1-methylethyl)ester **15b**

Man behandelt 250 mg des Phosphonsäureesters **12** analog 2a) und erhält 178 mg (5*Z*,7*E*,22*E*)-(1*S*,3*R*)-[1,3-Bis[[dimethyl(1,1-dimethylethyl)silyl]oxy]-24-hydroxy-24a-homo-9,10-secochola-5,7,10(19),22-tetraen-24a-yl]phosphonsäurebis(1-methylethyl)ester **14** als farblosen Schaum (1:1-Gemisch der Diastereomeren bzgl. C-24).

$^1$H-NMR (300 MHz, $CD_2Cl_2$): δ= 0,06 ppm (s, 12 H); 0,52 (s, 3H); 0,86 (s, 18H); 1,00/1,02 (jeweils d, 3H); 1,31 (d, 12H); 4,16 (m, 1H); 4,35 (m, 2H); 4,63 (hept, 2H); 4,83 (s, 1H); 5,16 (s, 1H); 5,37/5,38 (jeweils dd, 1H); 5,50/5,52 (jeweils dd, 1H); 6,00 (d, 1H); 6,22 (d, 1H)

b) Man behandelt 130 mg des Diastereomerengemisches **14** analog 1d) und trennt die Epimeren schließlich über HPLC, wobei man nacheinander 34 mg der Titelverbindung **15a** und 33 mg der Titelverbindung **15b** jeweils als farblosen Schaum erhält.

$^1$H-NMR (300 MHz, $CD_2Cl_2$): **15a**: δ= 0,56 ppm (s, 3 H); 1,03 (d, 3H); 1,31 (d, 12H); 4,18 (m, 1H); 4,38 (m, 2H); 4,68 (hept, 2H); 4,95 (s, 1H; 5,28 (s, 1H); 5,40 (dd, 1H); 5,56 (dd, 1H); 6,00 (d, 1H); 6,36 (d, 1H)

**15b**: δ=0,55 ppm (s, 3 H); 1,04 (d, 3H); 1,30 (d, 12H); 4,18 (m, 1H); 4,38 (m, 2H); 4,69 (hept, 2H); 4,95 (s, 1H); 5,29 (s, 1H); 5,39 (dd, 1H); 5,53 (dd, 1H); 6,00 (d, 1H); 6,35 (d, 1H)

**Beispiel 7**

(5*Z*,7*E*,22*E*,24*Z*)-(1*S*,3*R*)-Phosphorsäure[1,3-dihydroxy-24a-(diethoxy)phosphinyl-24a-homo-9,10-secochola-5,7,10(19),22,24-pentaen-24-yl]diethylester **17**

a) Man bereitet aus 0,16 ml Diisopropylamin und 0,48 ml *n*-Butyllithium-Lösung (1.6 M in Hexan) in 20 ml THF bei -78°C unter Stickstoff Lithiumdiisopropylamid (LDA) und tropft 650 mg des Ketons **8** hinzu. Man rührt 30 min bei dieser Temperatur und gibt dann 414 mg Diethylchlorphosphat hinzu. Es wird auf 0°C erwärmt und 1 h nachgerührt. Dann quencht man mit Natriumchlorid-Lösung, extrahiert mit Essigester, wäscht die organische Phase mit Natriumchlorid-Lösung, trocknet über Natriumsulfat und engt ein. Der Rückstand wird an Silicagel mit Essigester/Hexan chromatographiert, wobei 216 mg (5*Z*,7*E*,22*E*,24*Z*)-(1*S*,3*R*)-Phosphorsäure[1,3-bis[[dimethyl(1,1-dimethylethyl)silyl]oxy]-24a-(diethoxy)phosphinyl-24a-homo-9,10-secochola-5,7,10(19),22,24-pentaen-24-yl]diethylester **16** als farbloser Schaum anfallen.

$^1$H-NMR (300 MHz, $CD_2Cl_2$): δ= 0,05 ppm (s, 12 H); 0,56 (s, 3H); 0,86 (s, 18H); 1,07 (d, 3H); 1,28 (t, 6H); 1,31 (t, 6H); 4,07 (q, 4H); 4,22 (q, 4H); 4,17 (m, 1H); 4,37 (m, 1H); 4,84 (s, 1H); 5,15 (d, 1H); 5,18 (s, 1H); 5,96 (d, 1H); 6,00 (d, 1H); 6,22 (d, 1H); 6,28 (dd, 1H)

b) Man behandelt 105 mg des Phosphonsäureesters **16** analog 1d) und erhält 56 mg der Titelverbindung **17** als farblosen Schaum.

$^1$H-NMR (300 MHz, $CD_2Cl_2$): δ= 0,57 ppm (s, 3 H); 1,07 (d, 3H); 1,29 (t, 6H); 1,31 (t, 6H); 4,03 (q, 2H); 4,07 (q, 2H); 4,21 (q, 2H); 4,24 (q, 2H); 4,18 (m, 1H); 4,38 (m, 1H); 4,96 (s, 1H); 5,17 (d, 1H); 5,29 (s, 1H); 5,99 (d, 1H); 6,00 (d, 1H); 6,29 (dd, 1H); 6,33 (d, 1H)

**Beispiel 8**

(5*Z*,7*E*,22*E*,24*E*)-(1*S*,3*R*)-(1,3-Dihydroxy-24a-homo-9,10-secochola-5,7,10(19),22,24-pentaen-24-ayl)phosphonsäurediethylester **20**

a) Man legt 10 mg Natriumhydrid (80% in Paraffinöl) in 2 ml THF unter Stickstoff vor und kühlt auf 0°C. Nun tropft man 61 mg Tetraethylmethylenbisphosphonat hinzu und rührt 5 min bei dieser Temperatur. Anschließend werden 125 mg (5*Z*,7*E*,22*E*)-(1*S*,3*R*)-1,3-Bis[[dimethyl(1,1-dimethylethyl)silyl]oxy]-9,10-secochola-5,7,10(19),22-tetraen-24-al **18** (P 51405 Schering AG) in 1 ml THF zugetropft und 1 h bei Raumtemperatur gerührt. Man gibt dann Natriumchlorid-Lösung hinzu, extrahiert mit Essigester, wäscht die organische Phase mit Natriumchlorid-Lösung, trocknet über Natriumsulfat und entfernt das Solvens. Der Rückstand wird an Silicagel mit Essigester/Hexan chromatographiert, wobei man 89 mg (5*Z*,7*E*,22*E*)-(1*S*,3*R*)-[1,3-Bis[[dimethyl(1,1-dimethylethyl)silyl]oxy]-24a-homo-9,10-secochola-5,7,10(19),22,24-pentaen-24a-yl]phosphonsäurediethylester **19** als farblosen Schaum erhält.

$^1$H-NMR (300 MHz, $CDCl_3$): δ= 0,06 ppm (s, 12 H); 0,57 (s, 3H); 0,89 (s, 18H); 1,08 (d, 3H); 1,32 (t, 6H); 4,08 (q, 2H); 4,10 (q, 2H); 4,18 (m, 1H); 4,38 (m, 1H); 4,86 (s, 1H); 5,18 (s, 1H); 5,57 (dd, 1H); 5,95 (dd, 1H); 6,02 (d, 1H); 6,09 (dd, 1H); 6,24 (d, 1H); 7,07 (dd, 1H)

b) Man behandelt 60 mg des Phosphonsäureesters **19** analog 1d) und erhält 31 mg der Titelverbindung **20** als farblosen Schaum.

$^1$H-NMR (300 MHz, $CD_2Cl_2$): δ= 0,53 ppm (s, 3 H); 1,03 (d, 3H); 1,26 (t, 6H); 4,00 (q, 2H); 4,03 (q, 2H); 4,17 (m, 1H); 4,37 (m, 1H); 4,95 (s, 1H); 5,28 (s, 1H); 5,53 (dd, 1H); 5,94 (dd, 1H); 6,00 (d, 1H); 6,05 (dd, 1H); 6,34 (d, 1H); 6,95 (dd, 1H)

**Beispiel 9**

(5*Z*,7*E*,22*E*)-(1*S*,3*R*)-24-(Diphenylphosphinyl)-9,10-secochola-5,7,10(19),22-tetraen-1,3-diol **23**

a) Man legt 750 mg (5*Z*,7*E*,22*E*)-(1*S*,3*R*)-1,3-Bis[[dimethyl(1,1-dimethylethyl)silyl]oxy]-9,10-secochola-5,7,10(19),22-tetraen-24-al **18** in 20 ml Toluol unter Stickstoff vor und tropft bei -78°C 2,58 ml Diisobutylaluminium-Lösung (1.2 M in Toluol) hinzu. Nach 30 min bei dieser Temperatur werden 3 ml eines Wasser/Isopropanol-Gemisches (1:1) zugetropft und es wird 1 h nachgerührt. Der Niederschlag wird dann abfiltriert und das Filtrat eingeengt und durch Chromatographie an Silicagel mit Essigester/Hexan gereinigt, wobei man 550 mg (5*Z*,7*E*,22*E*)-(1*S*,3*R*)-1,3-Bis[[dimethyl(1,1-dimethylethyl)silyl]oxy]-9,10-secochola-5,7,10(19),22-tetraen-24-ol **21** als farblosen Schaum erhält.

$^1$H-NMR (300 MHz, $CDCl_3$): δ= 0,06 ppm (s, 12 H); 0,58 (s, 3H); 0,88 (s, 18H); 1,07 (d, 3H); 4,08 (m, 2H); 4,18 (m, 1H); 4,38 (m, 1H); 4,87 (s, 1H); 5,18 (s, 1H); 5,57 (m, 2H); 6,01 (d, 1H); 6,24 (d, 1H)

b) Man legt 80 mg des Alkohols **21** in 2 ml THF unter Stickstoff vor, kühlt auf 0°C und tropft 0,06 ml *n*-Butyllithium-Lösung (1.6 M in Hexan) zu und rührt 10 min nach. Nun werden 26 mg p-Toluolsulfonsäurechlorid in 1 ml THF zugegeben und erneut wird 10 min gerührt. Parallel wird eine Lösung von 25 mg Diphenylphosphin in 2 ml THF bei - 10°C unter Stickstoff mit 0,05 ml *n*-Butyllithium-Lösung (1.6 M in Hexan) behandelt und 10 min gerührt. Zu dieser Lösung tropft man das in situ generierte Tosylat und rührt 1 h bei Raumtemperatur nach. Man engt das Reaktionsgemisch ein und löst den Rückstand in 3 ml Chloroform. Es werden nun 0,5 ml Wasserstoffperoxid-Lösung (5% in Wasser) hinzugegeben und 2 h bei Raumtemperatur nachgerührt. Die Phasen werden getrennt, die organische Phase wird mit Natriumthiosulfat-Lösung gewaschen, über Natriumsulfat getrocknet und eingeengt. Der Rückstand wird an Silicagel mit Essigester/Hexan chromatographiert, wobei 58 mg (5*Z*,7*E*,22*E*)-(1*S*,3*R*)-1,3-Bis[[dimethyl(1,1-dimethylethyl)silyl]oxy]-24-(diphenylphosphinyl)-9,10-secochola-5,7,10(19),22-tetraen **22** als farbloser Schaum anfallen.

$^1$H-NMR (300 MHz, $CD_2Cl_2$): δ= 0,05 ppm (s, 12 H); 0,42 (s, 3H); 0,86 (s, 18H); 0,92 (d, 3H); 3,01 (d, 2H); 4,16 (m, 1H); 4,36 (m, 1H); 4,84 (s, 1H); 5,16 (s, 1H); 5,30 (m, 2H); 5,99 (d, 1H); 6,22 (d, 1H); 7,48 (m, 6H); 7,68 (m, 4H)

c) Man behandelt 34 mg des Phosphinoxids **22** analog 1d) und erhält 16 mg der Titelverbindung **23** als farblosen Schaum.

$^1$H-NMR (300 MHz, $CD_2Cl_2$): δ= 0,47 ppm (s, 3 H); 0,88 (d, 3H); 3,02 (d, 2H); 4,17 (m, 1H); 4,37 (m, 1H); 4,92 (s, 1H); 5,28 (s, 1H); 5,30 (m, 2H); 5,98 (d, 1H); 6,30 (d, 1H); 7,47 (m, 6H); 7,68 (m, 4H)

## Patentansprüche

1. Verbindungen der allgemeinen Formel **I**,

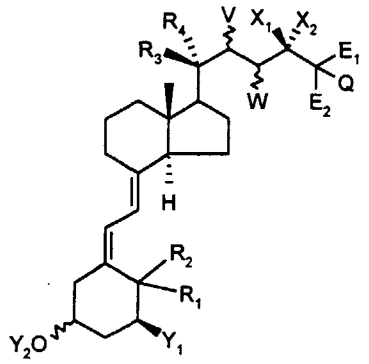

**I**

worin

$Y_1$ ein Wasserstoffatom, eine Hydroxylgruppe, ein Fluor-, Chlor- oder Bromatom oder eine Gruppe -O(CO)$R_5$, worin
$R_5$ ein aliphatischer oder aromatischer Rest mit 1 bis 12 C-Atomen ist,
$Y_2$ ein Wasserstoffatom oder eine Gruppe -(CO)$R_6$, worin
$R_6$ ein aliphatischer oder aromatischer Rest mit 1 bis 12 C-Atomen ist

und die Gruppe $Y_2$O sowohl in der natürlich vorkommenden (3β) als auch der epimeren (3α) Situation vorliegen kann,

$R_1$ und $R_2$ je ein Wasserstoffatom oder gemeinsam eine exocyclische Methylengruppe,
$R_3$ und $R_4$ unabhängig voneinander ein Wasserstoffatom, ein Chlor- oder Fluoratom, eine Alkylgruppe mit 1 bis 4 Kohlenstoffatomen, gemeinsam eine Methylengruppe oder gemeinsam mit dem quartären Kohlenstoffatom 20 einen 3-7-gliedrigen, gesättigten oder ungesättigten carbocyclischen Ring,
V und W zusammen eine *E*-Doppelbindung oder V eine Hydroxylgruppe und W ein Wasserstoffatom oder V und W jeweils Wasserstoffatome,
$X_1$ und $X_2$ gemeinsam ein Carbonylgruppe oder je ein Wasserstoffatom oder $X_2$ ein Wasserstoffatom und $X_1$ eine Hydroxylgruppe oder eine Gruppe -O(CO)$R_7$, worin
$R_7$ ein aliphatischer oder aromatischer Rest mit 1 bis 12 C-Atomen ist,
oder $X_1$ ein Wasserstoffatom und $X_2$ eine Hydroxylgruppe oder eine Gruppe -O(CO)$R_8$, worin
$R_8$ ein aliphatischer oder aromatischer Rest mit 1 bis 12 C-Atomen ist,
oder
$X_1$ und $E_2$ eine Doppelbindung und gleichzeitig $X_2$ ein Wasserstoffatom oder eine Gruppe O-Z, wobei Z ein aliphatischer oder aromatischer Rest mit 1 bis 12 C-Atomen, eine aliphatische oder aromatische Acylgruppe mit 1 bis 12 C-Atomen oder eine Gruppe $E_2$,
$E_1$ eine Gruppe $PO(OR_9)_2$, eine Gruppe $PO(N(R_9)_2)_2$, eine Gruppe $PO(R_9)_2$ oder eine Gruppe $CO_2R_9$ worin
$R_9$ ein Wasserstoffatom oder ein aliphatischer oder aromatischer Rest mit 1 bis 12C-Atomen ist,
$E_2$ eine Gruppe $PO(OR_9)_2$, eine Gruppe $PO(N(R_9)_2)_2$, eine Gruppe $PO(R_9)_2$, eine Gruppe $CO_2R_9$ oder ein Wasserstoffatom,
Q ein Wasserstoffatom, ein aliphatischer oder aromatischer Rest mit 1 bis 12 C-Atomen oder eine Hydroxylgruppe, eine Gruppe -O(CO)$R_{10}$, ein Fluor, Chlor oder Bromatom, eine Aminogruppe oder eine Gruppe $NHR_{10}$ oder $N(R_{10})_2$, worin
$R_{10}$ ein aliphatischer oder aromatischer Rest mit 1 bis 12 C-Atomen ist,

bedeuten.

**2.** Verbindungen gemäß Anspruch 1, dadurch gekennzeichnet, daß mindestens eine phosphorhaltige Gruppe enthalten ist.

**3.** Verbindungen gemaß Anspruch 1, dadurch gekennzeichnet, daß $E_1$ und $E_2$ für Phosphonsäurederivate stehen.

**4.** Verbindungen gemäß Anspruch 1, dadurch gekennzeichnet, daß die Gruppen $R_3$ und $R_4$ für folgende Reste stehen:

$R_3$= H, $R_4$= Methyl oder
$R_3$= Methyl, $R_4$=H oder
$R_3$= F, $R_4$= Methyl oder
$R_3$= Methyl,$R_4$=F oder
$R_3$= Methyl, $R_4$= Methyl oder
$R_3$ und $R_4$ bilden zusammen eine Methylengruppe oder
$R_3$ und $R_4$ bilden gemeinsam mit dem tertiären Kohlenstoffatom 20 einen Cyclopropylring.

**5.** Verbindungen gemäß Anspruch 1, dadurch gekennzeichnet, daß V und W und $X_1$ und $E_2$ jeweils eine *E*-Doppelbindung bedeuten, während $X_2$ und Q Wasserstoffatome sind.

6. Verbindungen gemäß Anspruch 1, dadurch gekennzeichnet, daß V und W zusammen eine *E*-Doppelbindung bilden, $X_1$ und $E_2$ eine *E*- oder *Z*-Doppelbindung bilden, Q ein Wasserstoffatom bedeutet, $X_2$ eine Gruppe -OZ ist, worin Z die in Anspruch 1 genannte Bedeutung hat.

7. Verbindungen gemäß Anspruch 1, nämlich:

(5*Z*,7*E*,22*E*)-(1*S*,3*R*)-(1,3-Dihydroxy-24-oxo-24a-homo-9,10-secochola-5,7,10(19),22-tetraen-24a-yl)phosphonsäuredimethylester,
(5*Z*,7*E*,22*E*)-(1*S*,3*R*,24*S*)-(1,3,24-Trihydroxy-24a-homo-9,10-secochola-5,7,10(19),22-tetraen-24a-yl)phosphonsäuredimethylester,
(5*Z*,7*E*,22*E*)-(1*S*,3*R*,24*R*)-(1,3,24-Trihydroxy-24a-homo-9,10-secochola-5,7,10(19),22-tetraen-24a-yl)phosphonsäuredimethylester,
(5*Z*,7*E*,22*E*)-(1*S*,3*R*)-(1,3-Dihydroxy-24-oxo-24a-homo-9,10-secochola-5,7,10(19),22-tetraen-24a-yl)phosphonsäurediethylester,
(5*Z*,7*E*,22*E*)-(1*S*,3*R*,24*S*)-(1,3,24-Trihydroxy-24a-homo-9,10-secochola-5,7,10(19),22-tetraen-24a-yl)phosphonsäurediethylester,
(5*Z*,7*E*,22*E*)-(1*S*,3*R*,24*R*)-(1,3,24-Trihydroxy-24a-homo-9,10-secochola-5,7,10(19),22-tetraen-24a-yl)phosphonsäurediethylester,
(5*Z*,7*E*,22*E*)-(1*S*,3*R*)-(1,3-Dihydroxy-24-oxo-24a-homo-9,10-secochola-5,7,10(19),22-tetraen-24a-yl)phosphonsäurebis(1-methylethyl)ester,
(5*Z*,7*E*,22*E*)-(1*S*,3*R*,24*S*)-(1,3,24-Trihydroxy-24a-homo-9,10-secochola-5,7,10(19),22-tetraen-24a-yl)phosphonsäurebis(1-methylethyl)ester,
(5*Z*,7*E*,22*E*)-(1*S*,3*R*,24*R*)-(1,3,24-Trihydroxy-24a-homo-9,10-secochola-5,7,10(19),22-tetraen-24a-yl)phosphonsäurebis(1-methylethyl)ester,
(5*Z*,7*E*,22*E*)-(1*S*,3*R*)-(1,3-Dihydroxy-24-oxo-24a-homo-9,10-secochola-5,7,10(19),22-tetraen-24a-yl)phosphonsäuredipropylester,
(5*Z*,7*E*,22*E*)-(1*S*,3*R*,24*S*)-(1,3,24-Trihydroxy-24a-homo-9,10-secochola-5,7,10(19),22-tetraen-24a-yl)phosphonsäuredipropylester,
(5*Z*,7*E*,22*E*)-(1*S*,3*R*,24*R*)-(1,3,24-Trihydroxy-24a-homo-9,10-secochola-5,7,10(19),22-tetraen-24a-yl)phosphonsäuredipropylester,
(5*Z*,7*E*,22*E*)-(1*S*,3*R*)-(1,3-Dihydroxy-24-oxo-24a-homo-9,10-secochola-5,7,10(19),22-tetraen-24a-yl)phosphonsäuredibutylester,
(5*Z*,7*E*,22*E*)-(1*S*,3*R*,24*S*)-(1,3,24-Trihydroxy-24a-homo-9,10-secochola-5,7,10,(19),22-tetraen-24a-yl)phosphonsäuredibutylester,
(5*Z*,7*E*,22*E*)-(1*S*,3*R*,24*R*)-(1,3,24-Trihydroxy-24a-homo-9,10-secochola-5,7,10(19),22-tetraen-24a-yl)phosphonsäuredibutylester,
(5*Z*,7*E*,22*E*)-(1*S*,3*R*)-(1,3-Dihydroxy-24-oxo-24a-homo-9,10-secochola-5,7,10(19),22-tetraen-24a-yl)phosphonsäuredipentylester,
(5*Z*,7*E*,22*E*)-(1*S*,3*R*,24*S*)-(1,3,24-Trihydroxy-24a-homo-9,10-secochola-5,7,10(19),22-tetraen-24a-yl)phosphonsäuredipentylester,
(5*Z*,7*E*,22*E*)-(1*S*,3*R*,24*R*)-(1,3,24-Trihydroxy-24a-homo-9,10-secochola-5,7,10(19),22-tetraen-24a-yl)phosphonsäuredipentylester,
(5*Z*,7*E*,22*E*)-(1*S*,3*R*)-(1,3-Dihydroxy-24-oxo-24a-homo-9,10-secochola-5,7,10(19),22-tetraen-24a-yl)phosphonsäurediphenylester,
(5*Z*,7*E*,22*E*)-(1*S*,3*R*,24*S*)-(1,3,24-Trihydroxy-24a-homo-9,10-secochola-5,7,10(19),22-tetraen-24a-yl)phosphonsäurediphenylester,
(5*Z*,7*E*,22*E*)-(1*S*,3*R*,24*R*)-(1,3,24-Trihydroxy-24a-homo-9,10-secochola-5,7,10(19),22-tetraen-24a-yl)phosphonsäurediphenylester,
(5*Z*,7*E*,22*E*)-(1*S*,3*R*)-(1,3-Dihydroxy-24-oxo-24a-homo-9,10-secochola-5,7,10(19),22-tetraen-24a,24a-diyl)bis[phosphonsäuredimethylester],
(5*Z*,7*E*,22*E*)-(1*S*,3*R*)-(1,3-Dihydroxy-24-oxo-24a-homo-9,10-secochola-5,7,10(19),22-tetraen-24a,24a-diyl)bis[phosphonsäurediethylester],
(5*Z*,7*E*,22*E*)-(1*S*,3*R*)-(1,3-Dihydroxy-24-oxo-24a-homo-9,10-secochola-5,7,10(19),22-tetraen-24a,24a-diyl)bis[phosphonsäuredipropylester],
(5*Z*,7*E*,22*E*)-(1*S*,3*R*)-(1,3-Dihydroxy-24-oxo-24a-homo-9,10-secochola-5,7,10(19),22-tetraen-24a,24a-diyl)bis[phosphonsäuredibutylester],
(5*Z*,7*E*,22*E*)-(1*S*,3*R*)-(1,3-Dihydroxy-24-oxo-24a-homo-9,10-secochola-5,7,10(19),22-tetraen-24a,24a-diyl)bis[phosphonsäurebis(1-methylethyl)ester],

(5*Z*,7*E*,22*E*)-(1*S*,3*R*)-(1,3-Dihydroxy-24-oxo-24a-homo-9,10-secochola-5,7,10(19),22-tetraen-24a,24a-diyl)bis[phosphonsäurediphenylester],
(5*Z*,7*E*,22*E*,24*E*)-(1*S*,3*R*)-(1,3-Dihydroxy-24a-homo-9,10-secochola-5,7,10(19),22,24-pentaen-24a-yl)phosphonsäuredimethylester,
(5*Z*,7*E*,22*E*,24*E*)-(1*S*,3*R*)-(1,3-Dihydroxy-24a-homo-9,10-secochola-5,7,10(19),22,24-pentaen-24a-yl)phosphonsäurediethylester,
(5*Z*,7*E*,22*E*,24*E*)-(1*S*,3*R*)-(1,3-Dihydroxy-24a-homo-9,10-secochola-5,7,10(19),22,24-pentaen-24a-yl)phosphonsäuredipropylester,
(5*Z*,7*E*,22*E*,24*E*)-(1*S*,3*R*)-(1,3-Dihydroxy-24a-homo-9,10-secochola-5,7,10(19),22,24-pentaen-24a-yl)phosphonsäuredibutylester,
(5*Z*,7*E*,22*E*,24*E*)-(1*S*,3*R*)-(1,3-Dihydroxy-24a-homo-9,10-secochola-5,7,10(19),22,24-pentaen-24a-yl)phosphonsäurebis(1-methylethyl)ester,
(5*Z*,7*E*,22*E*)-(1*S*,3*R*)-24-(Diphenylphosphinyl)-9,10-secochola-5,7,10(19),22-tetraen-1,3-diol,
(5*Z*,7*E*,22*E*)-(1*S*,3*R*)-24-(Dimethylphosphinyl)-9,10-secochola-5,7,10(19),22-tetraen-1,3-diol,
(5*Z*,7*E*,22*E*)-(1*S*,3*R*)-24-(Diethylphosphinyl)-9,10-secochola-5,7,10(19),22-tetraen-1,3-diol,
(5*Z*,7*E*,22*E*)-(1*S*,3*R*)-24-(Dipropylphosphinyl)-9,10-secochola-5,7,10(19),22-tetraen-1,3-diol,
(5*Z*,7*E*,22*E*)-(1*S*,3*R*)-24-(Dibutylphosphinyl)-9,10-secochola-5,7,10(19),22-tetraen-1,3-diol,
(5*Z*,7*E*,22*E*,24*E*)-(1*S*,3*R*)-Phosphorsäure[1,3-dihydroxy-24a-(dimethoxy)phosphinyl-24a-homo-9,10-secochola-5,7,10(19),22,24-pentaen-24-yl]dimethylester,
(5*Z*,7*E*,22*E*,24*Z*)-(1*S*,3*R*)-Phosphorsäure[1,3-dihydroxy-24a-(dimethoxy)phosphinyl-24a-homo-9,10-secochola-5,7,10(19),22,24-pentaen-24-yl]dimethylester,
(5*Z*,7*E*,22*E*,24*E*)-(1*S*,3*R*)-Phosphorsäure[24a-(diethoxy)phosphinyl-1,3-dihydroxy-24a-homo-9,10-secochola-5,7,10(19),22,24-pentaen-24-yl]diethylester,
(5*Z*,7*E*,22*E*,24*Z*)-(1*S*,3*R*)-Phosphorsäure[24a-(diethoxy)phosphinyl-1,3-dihydroxy-24a-homo-9,10-secochola-5,7,10(19),22,24-pentaen-24 yl]diethylester,
(5*Z*,7*E*,22*E*,24*E*)-(1*S*,3*R*)-Phosphorsäure[1,3-dihydroxy-24a-(dipropoxy)phosphinyl-24a-homo-9,10-secochola-5,7,10(19),22,24-pentaen-24-yl]dipropylester,
(5*Z*,7*E*,22*E*,24*Z*)-(1*S*,3*R*)-Phosphorsäure[1,3-dihydroxy-24a-(dipropoxy)phosphinyl-24a-homo-9,10-secochola-5,7,10(19),22,24-pentaen-24 yl]dipropylester,
(5*Z*,7*E*,22*E*,24*E*)-(1*S*,3*R*)-Phosphorsäure[24a-(dibutoxy)phosphinyl-1,3-dihydroxy-24a-homo-9,10-secochola-5,7,10(19),22,24-pentaen-24-yl]dibutylester,
(5*Z*,7*E*,22*E*,24*Z*)-(1*S*,3*R*)-Phosphorsäure[24a-(dibutoxy)phosphinyl-1,3-dihydroxy-24a-homo-9,10-secochola-5,7,10(19),22,24-pentaen-24-yl]dibutylester,
(5*Z*,7*E*,22*E*,24*E*)-(1*S*,3*R*)-Phosphorsäure(24a-(bis(1-methylethoxy)phosphinyl]-1,3-dihydroxy-24a-homo-9,10-secochola-5,7,10(19),22,24-pentaen-24-yl]bis(1-methylethyl)ester,
(5*Z*,7*E*,22*E*,24*Z*)-(1*S*,3*R*)-Phosphorsäure[24a-[bis(1-methylethoxy)phosphinyl]-1,3-dihydroxy-24a-homo-9,10-secochola-5,7,10(19),22,24-pentaen-24-yl]bis(1-methylethyl)ester.

**8.** Verwendung von Verbindungen gemäß mindestens einem der Ansprüche 1-7 zur Herstellung eines Arzneimittels.

**9.** Verwendung von Verbindungen gemäß mindestens einem der Ansprüche 1-7 zur Herstellung eines Arzneimittels zur Therapie von Osteoporose.

**10.** Verbindung der allgemeinen Formel **II**,

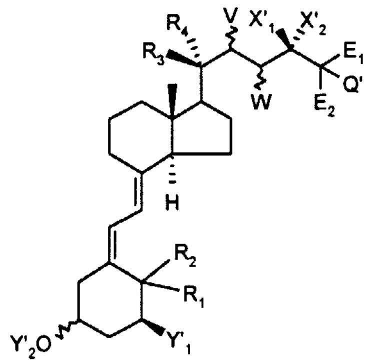

II

worin

$Y'_1$ ein Wasserstoffatom, eine geschützte Hydroxylgruppe, ein Fluor-, Chlor- oder Bromatom oder eine Gruppe $-O(CO)R_5$, worin
$R_5$ ein aliphatischer oder aromatischer Rest mit 1 bis 12 C-Atomen ist,

$Y'_2$ ein Wasserstoffatom oder eine Gruppe $-(CO')R_6$, worin
(CO') eine geschützte Ketogruppe ist $R_6$ ein aliphatischer oder aromatischer Rest mit 1 bis 12 C-Atomen ist

und die Gruppe $Y'_2O$ sowohl in der natürlich vorkommenden (3β) als auch der epimeren (3α) Situation vorliegen kann,

$R_1$ und $R_2$ je ein Wasserstoffatom oder gemeinsam eine exocyclische Methylengruppe,

$R_3$ und $R_4$ unabhängig voneinander ein Wasserstoffatom, ein Chlor- oder Fluoratom, eine Alkylgruppe mit 1 bis 4 Kohlenstoffatomen, gemeinsam eine Methylengruppe oder gemeinsam mit dem quartären Kohlenstoffatom 20 einen 3-7-gliedrigen, gesättigten oder ungesättigten carbocyclischen Ring,

V und W zusammen eine *E*-Doppelbindung oder V eine Hydroxylgruppe und W ein Wasserstoffatom oder V und W jeweils Wasserstoffatome,

$X'_1$ und $X'_2$ gemeinsam eine geschützte Carbonylgruppe oder je ein Wasserstoffatom oder $X'_2$ ein Wasserstoffatom und $X'_1$ eine geschützte Hydroxylgruppe oder eine Gruppe $-O(CO)R_7$, worin
$R_7$ ein aliphatischer oder aromatischer Rest mit 1 bis 12 C-Atomen ist,
oder $X_1$ ein Wasserstoffatom und $X_2$ eine geschützte Hydroxylgruppe oder eine Gruppe $-O(CO)R_8$, worin
$R_8$ ein aliphatischer oder aromatischer Rest mit 1 bis 12 C-Atomen ist,
oder

$X_1$ und $E_2$ eine Doppelbindung und gleichzeitig $X_2$ ein Wasserstoffatom oder eine Gruppe O-Z,
wobei Z ein aliphatischer oder aromatischer Rest mit 1 bis 12 C-Atomen, eine aliphatische oder aromatische Acylgruppe mit 1 bis 12 C-Atomen oder eine Gruppe $E_2$,

$E_1$ eine Gruppe $PO(OR_9)_2$, eine Gruppe $PO(N(R_9)_2)_2$, eine Gruppe $PO(R_9)_2$ oder eine Gruppe $CO_2R_9$ worin
$R_9$ ein Wasserstoffatom oder ein aliphatischer oder aromatischer Rest mit 1 bis 12C-Atomen ist,

$E_2$ eine Gruppe $PO(OR_9)_2$, eine Gruppe $PO(N(R_9)_2)_2$, eine Gruppe $PO(R_9)_2$, eine Gruppe $CO_2R_9$ oder ein Wasserstoffatom,

Q ein Wasserstoffatom, ein aliphatischer oder aromatischer Rest mit 1 bis 12 C-Atomen oder eine geschützte Hydroxylgruppe, eine Gruppe $-O(CO)R_{10}$, ein Fluor-, Chlor- oder Bromatom, eine geschützte Aminogruppe oder eine gegebenenfalls geschützte Gruppe $NHR_{10}$ oder $N(R_{10})_2$, worin $R_{10}$ ein aliphatischer oder aromatischer Rest mit 1 bis 12 C-Atomen ist,

bedeuten.

**11.** Verbindung gemäß Anspruch 10, dadurch gekennzeichnet, daß X1' und X2' gemeinsam eine geschützte Carbonylgruppe bilden, V und W eine *E*-Doppelbindung bilden und $E_2$ und Q' Wasserstoffatome sind.

Figur 1/1:

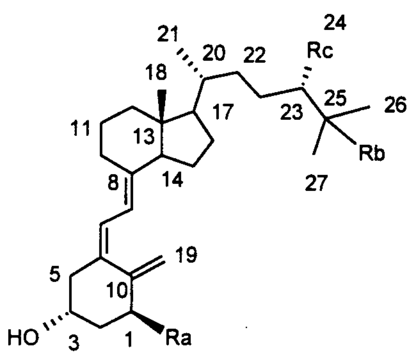

| | | |
|---|---|---|
| Ergocalciferol | Ra=Rb=H, Rc=$CH_3$,<br>Doppelbindung C-22/23 | Vitamin $D_2$ |
| Cholecalciferol | Ra=Rb=Rc=H | Vitamin $D_3$ |
| 25-Hydroxycholecalciferol | Ra=Rc=H, Rb=OH | |
| 1α-Hydroxycholecalciferol | Ra=OH, Rb=Rc=H | |
| 1α,25-Dihydroxycholecalciferol | Ra=Rb=OH, Rc=H | Calcitriol |

Europäisches Patentamt

# EUROPÄISCHER RECHERCHENBERICHT

Nummer der Anmeldung
EP 97 25 0374

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,Y | WO 94 07853 A (SCHERING AG) 14.April 1994<br>* das ganze Dokument *<br>--- | 1-11 | C07F9/40<br>A61K31/66<br>C07F9/53<br>C07C401/00 |
| D,Y | WO 97 00242 A (SCHERING AG) 3.Januar 1997<br>* das ganze Dokument *<br>--- | 1-11 | |
| X | DE 43 36 429 A (SCHERING AG)<br>* das ganze Dokument *<br>--- | 10,11 | |
| X | EP 0 633 245 A (DUPHAR INTERNATIONAL RESEARCH B.V.) 11.Januar 1995<br>* das ganze Dokument *<br>--- | 10,11 | |
| A | US 4 230 700 A (MARION D. FRANCIS) 28.Oktober 1980<br>* das ganze Dokument *<br>--- | 1-9 | |
| A | WO 90 09992 A (LEO PHARMACEUTICAL PRODUCTS LTD.) 7.September 1990<br>* das ganze Dokument *<br>----- | 1-9 | |

| RECHERCHIERTE SACHGEBIETE (Int.Cl.6) |
|---|
| C07F<br>C07C<br>A61K |

Der vorliegende Recherchenbericht wurde für alle Patentansprüche erstellt

| Recherchenort | Abschlußdatum der Recherche | Prüfer |
|---|---|---|
| DEN HAAG | 19.Mai 1998 | Beslier, L |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)